# EUROPEAN PATENT SPECIFICATION

(11) **EP 0 869 801 B1**
(45) Date of publication and mention of the grant of the patent: **21.01.2004**
(21) Application number: 96942049.6
(22) Date of filing: 21.11.1996
(51) Int. Cl.: A61K 35/12

(54) **THERAPEUTIC AND DIAGNOSTIC AGENTS FOR THE TREATMENT OF MICROBIAL INFECTIONS**
THERAPEUTISCHE UND DIAGNOSTISCHE AGENZIEN ZUR BEHANDLUNG MIKROBIELLER INFEKTIONEN
AGENTS THERAPEUTIQUES ET DE DIAGNOSTIC POUR TRAITER LES INFECTIONS MICROBIENNES

(30) Priority: 22.11.1995 US 7477 P
(43) Date of publication of application: 14.10.1998
(73) Proprietor: Montana State University, Bozeman, MT 59717 (US); Ligocyte Pharmaceuticals, Inc., Bozeman, MT 59718 (US)
(72) Inventor: PASCUAL, David, Bozeman, MT 59178 (US); BURRITT, James, Bozeman, MT 59715 (US); BURGESS, Don, Bozeman, MT 59715 (US); GLEE, Pati, Bozeman, MT 59718 (US); JUTILA, John, Bozeman, MT 59715 (US); JUTILA, Mark, Bozeman, MT 59715 (US); BARGATZE, Robert, Bozeman, MT 59715 (US); PYLE, Barry, Bozeman, MT 59175 (US); CUTLER, Jim, E., Bozeman, MT 59715 (US); HAN, Yongmoon, Bozeman, MT 59715 (US)
(74) Representative: Gervasi, Gemma, Dr.
(86) International application number: PCT/US1996/018796
(87) International publication number: WO 1997/018790

(56) References cited:
- EP-A- 0 391 088
- WO-A-84/01290
- WO-A-92/02817
- WO-A-94/05269
- US-A- 5 081 034
- GLYCOCONJUGATE JOURNAL, Volume 11, issued 1994, SANDROS et al., "Lectin Domains in the Toxin of Bordetella Pertussis: Selectin Mimicry Linked to Microbial Pathogenesis", pages 501-506.

## Description

### Technical Field

The present invention relates to therapeutic peptides and carbohydrates, vaccines and diagnostic agents for the treatment of microbial infections, and is characterized as adhesion blocking technology.

### Background

In infectious disease, a pathogen traffics or migrates from one location to another, in a manner analogous to leukocytes engaging in their movement through the body. The similarity of leukocyte and microbial trafficking events, most notably molecular address recognition, signal transduction, and passage through endothelia and epithelia, implies that many microbial adhesion and signaling systems are evolutionary products of functional and molecular mimicry of host protein and carbohydrate moieties. This convergence of cell biology and microbial pathogenesis predicts that for each system of mammalian cell trafficking, a pathogen exists that is intimately familiar with the network.

A pathogen's ability to infect a host should no longer be defined solely in terms of invasive or toxigenic properties that simply kill cells and/or overwhelm the immune mechanisms of the host. Instead, recent studies (1,2,3,27,2C,44,52) suggest that the pathogenicity of a microbe may be more accurately defined in terms cf their ability to exploit the host's cellular communications network as a means of finding cellular and tissue environments favorable to the growth and proliferation of the pathogen. Thus, the pathogen minimizes the insult to the host while exploiting existing modes of trafficking in the complex chemical and cellular environments of the host. In functional terms, the travel of pathogen to tissue environments of choice which underlies the phenomenon of tissue tropism may be viewed as the consequence of a pathogen's recognition of host cells or extracellular matrix bearing a specific molecular address. Other microbial adhesion factors function to signal host cells to accommodate entry or passage of the pathogen into the cell or surrounding stroma. In other host-parasite interactions, the pathogen deploys intra-cellular signals that preempt or redirect the host's metabolic machinery in support of the growth and proliferation of the pathogen. These feats appear to be accomplished by the pathogen's use of the cell-cell communication network involving adhesion molecules of leukocytes, endothelial cells, epithelial cells and other target cells of the host.

United States patent applications having U.S. serial No. 08/247, 972 (related to PCT/US95/06832) and Serial No. 08/483,558 discloses a vaccine for the treatment of candidiasis comprising adhesion molecules of *Candida albicans* which are isolated phosphomannoprotein cell wall complexes in a liposome carrier.

The prior art has not recognized that pathogens and infectious agents use the host cell's own machinery (recognition and signaling systems) to mimic the host cell and enable the organism to traffic within the host and enter cells and tissue with relative ease. The present inventors have discovered pathogens use host cell universal antigens (attachment or adhesion molecules) to attach and proliferate. The invention overcomes difficulties in the prior art to develop a universal type vaccine for the treatment of pathogenic infections.

### Disclosure of the Invention

An object of the present invention is to provide a vaccine comprising a microbe targeting and attachment molecule adhesin molecule which mimics a host cell molecular address such as adhesion proteins, lipid molecules or carbohydrate molecules.

The invention provides a prophylactic and therapeutic vaccine comprising one or more attachment molecules or fragments thereof, capable of binding to a molecular address on a host cell, the binding capable of triggering one or more signal transduction pathways and enabling a selected pathogen and/or its toxin to traffic through host tissue.

Another object of the invention is to provide a vaccine comprising a microbial attachment molecule adhesin molecule which mimics host cell adhesion proteins or carbohydrate molecules of a cell selected from the group consisting of leukocytes, endothelial cells, and epithelial cells. The invention also provides for a vaccine comprising a microbial attachment molecule adhesin molecule.

In an alternative embodiment, the invention provides a diagnostic assay which comprises a monoclonal antibody specific for a microbial attachment molecule adhesin molecule which mimics host cell (adhesion) protein or carbohydrate molecules of a cell selected from the group consisting of leukocytes, endothelial cells, epithelial cells and other target cells of the hose.

Advantageously, the invention provides a therapeutic peptide or oligo peptide comprising a molecule which mimics the adhesin molecule of a pathogen and interacts with receptor molecules of a cell selected from the group consisting of leukocytes, endothelial cells, epithelial cells and other target cells of the host. The therapeutic peptide molecule may react specifically with receptor (ligands) of host adhesion molecules. Conversely, the invention provides a therapeutic peptide or carbohydrate comprising a molecule which mimics the adhesion molecule of a host cell and interacts with (blocks) the adhesin molecules of a pathogen.

The invention provides a method of obtaining a vaccine for development of immunity to a pathogen comprising the steps of
(a) selecting a pathogen adhesin molecule or fragments thereof that specifically binds to an adhesion molecule on a host cell or extracellular matrix;
(b) developing one or more monoclonal antibodies (mAbs) directed against at least one region of the isolated adhesin molecule or fragments thereof;
(c) isolating epitopes bound by said mAbs; and
(d) screening said selected epitopes to identify a molecule that induces a therapeutically effective imnune response against the pathogen.

The above and other objects of the invention will become readily apparent to those of skill in the relevant art from the following detailed description and figures, wherein only the preferred embodiments of the invention are shown and described, simply by way of illustration of the best mode of carrying out the invention. As is readily recognized the invention is capable of modifications within the skill of the relevant art without departing from the spirit and scope of the invention.

### Brief Description of Drawings

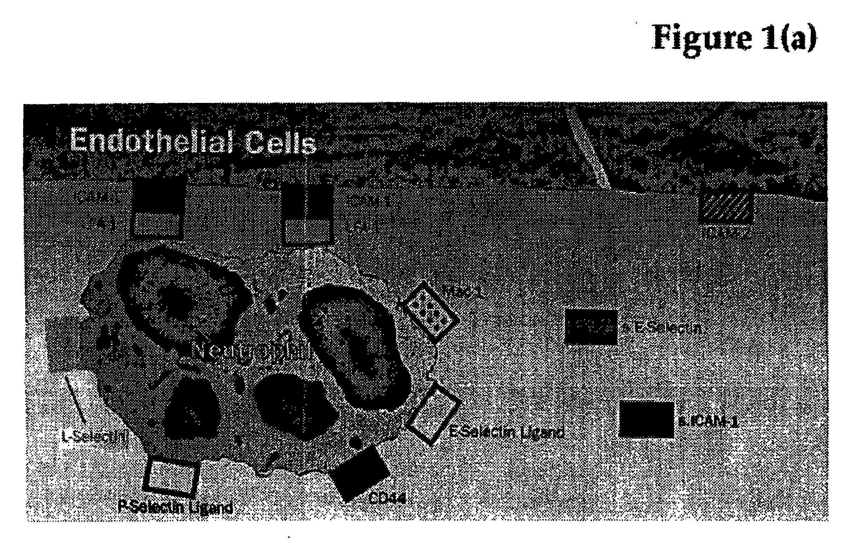

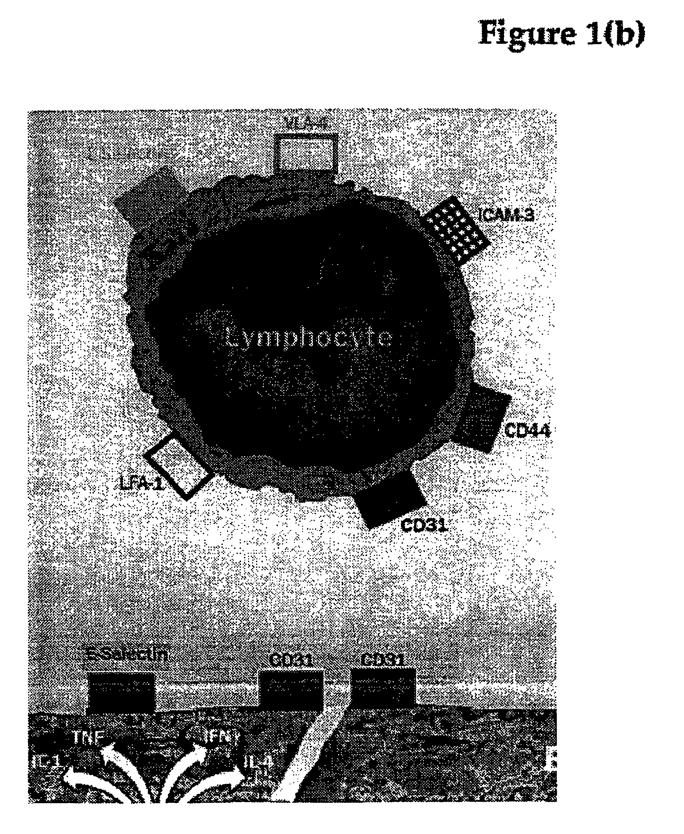

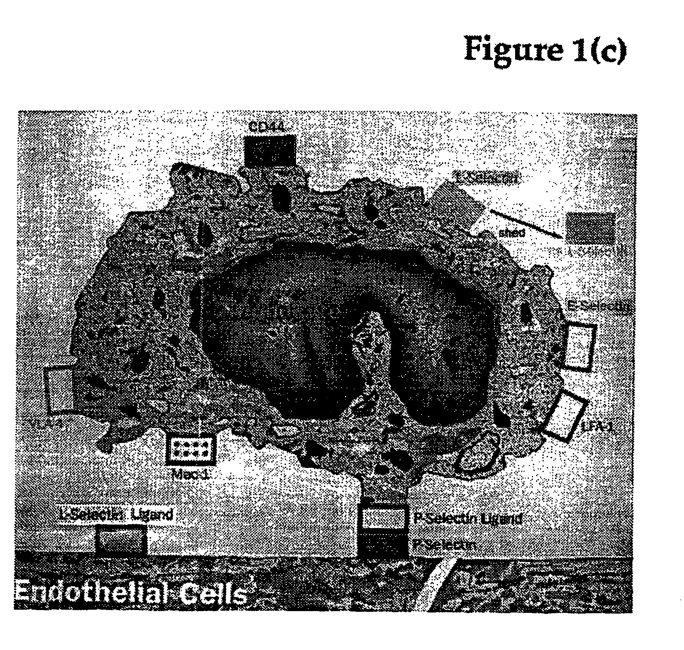

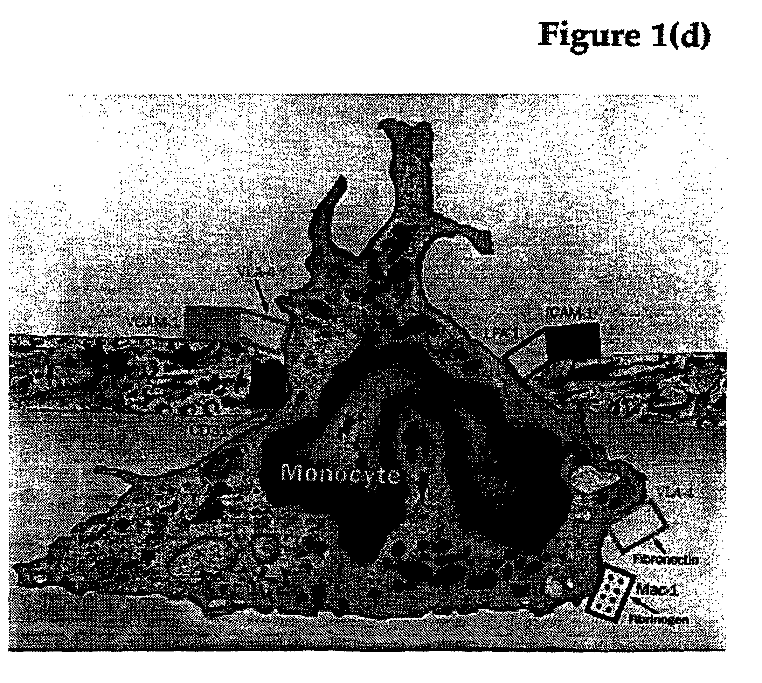

Figure 1 shows, a schematic diagram of a host/adhesion system. 1(a) shows a neutrophil host cell with attachment molecules; 1(b) shows a lymphocyte host cell with attachment molecules; 1(c) shows a monocyte host cell with attachment molecules; 1(d) shows endothelial host cells with attachment molecules.
Figure 2 shows a schematic of the prototype In vitro blood vessel shear flow system. An in vivo shear model employing intravital microscopy has also been developed by the inventors to examine cellular interactions under physiological shear forces found in the venules of the intact animal.

### Description of the Invention

The present invention relates to therapeutic peptides and carbohydrates, vaccines and diagnostic agents for the treatment of microbial infections. Generally, a vaccine in accordance with the invention comprises a microbial attachment molecule adhesin molecule. Vaccines in accordance with the invention more specifically comprises a microbial adhesin molecule or fragments thereof which mimic target host cell adhesion proteins or carbohydrate molecules.

In a preferred embodiment, the microbial adhesin molecule which mimics host cell adhesion proteins or carbohydrate molecules, mimics a host cell selected from the group consisting of leukocytes, endothelial cells, and epithelial cells.

The attachment molecule may resemble a selecting such as a molecule substantially and functionally similar to a selecting. In a preferred embodiment the selectin molecule is selected from those similar to E, L and P selectins,

In an alternative embodiment of the vaccine of the invention, the attachment molecule resembles an integrin, preferably VLA, Leucam and cytoadhesion family of integrins. In addition, molecules which are substantially and functionally similar to integrin molecules may be used, and more preferred are integrin molecules selected from VLA1-6, MAC-1, LFA1-3, CD41a, CD49 and CD51 integrins.

In an alternative embodiment, the attachment. molecule resembles a member of the immunoglobulin superfamily, preferably ICAMI-3, VCAM, NCAM and PECAM of the immunoglobulin superfamily. Molecules which are substantially and functionally similar to immunoglobulin superfamily molecules may also be used in the practice of the invention.

For an alternate embodiment, the attachment molecule resembles a membrane of the cytokine or chemokine family.

In accordance with the vaccine of the invention the attachment receptor molecule alternatively may resemble a mucin molecule or a prokaryotic or eukaryotic adhesion protein.

In the vaccine of the invention, the host cell adhesion proteins or carbohydrate molecules mimicked by the pathogen are of cells selected from the group consisting of leukocytes, endothelial cells, epithelial cells and cells of the nervous system. In a preferred embodiment, endothelial cells are selected from cytokine stimulated endothelial cells, such as ICAM-1 and VCAM-1 positive gut endothelial cell, MAdCAM-1 positive gut endothelial cells and PNAd-1 positive peripheral lymph node endothelial cells. The target host cell may be a respiratory pathway cell selected from the group consisting of cells of the nasopharynx and alveoli, including the alveolar macrophage.

The microbe supplying the microbial attachment molecule for the vaccine may be selected from microbes including *Bordetella pertussis, Plasmodium berghei, Plasmodium falciparum, Candida albi cans, Shigella sp., Entamoeba histolytica, Vibrio cholerae* and *Salmonella sp., E. coli* 0157:H7. The microbe may be alternatively selected from mycobacteria, *Legionella, staphylocci,* streptococci, histoplasma and Pneumococci. The microbe may be *Yersinia pseudotuberculosis* or *Yersinia enterocolitica*.

When the microbe is *Bordetella pertussis* the attachment molecule is preferably pertussis toxin and filamentous remaglutinin (FHA) or functionally equivalent molecules. The *Bordetella pertussis* host cell recognition molecules are encoded by protein subunits S2 and S3. When the microbe is *Helicobacter pylori* the attachment molecule preferably adheres to gastric epithelial cells by interacting with fucosylated blood group O phenotype antigens.

The microbe may alternatively be selected from *Trichomonas vaginales, Tritrichomonas foetus, Cryptosporidium*, *Giardia* and *Entamoeba.* The attachment molecule may be gp 63 of *Leishmania.*

Thus a vaccine of the invention is for the treatment of a microbial infection selected from among others, histoplasmosis, pulmonary blastomycosis, nocardiosis, cryptococcoses, bronchopulmonary candidiasis, pulmonary aspergillosis, tuberculosis, infections and Legionnaires disease.

In the vaccine of the invention, the adhesion molecule preferably mimics a CD18 adhesion selected from the group consisting of LFA-1, Mac-1 and P150.954, which recognize ligands on inflamed endothelia. The adhesion may be a 19 kDa EAF fimbrial bundle forming pilus adhesion expressed by a plasmid gene or associated with a bundle forming pilus from a bacterial strain selected from the group consisting of *Vibrio cholerae, Neisseria gonnorhoeae, Neisseria meningititis, Pseudomonas aeruginosa* and *Escherichia coli*. The adhesion of enteric microbes may alternatively be an outer membrane protein, chromosome encoded 94 kDa intimin which adheres to the epithelial cell plasma membrane. In another embodiment of the invention, the adhesion receptor is a polysialic acid, preferably having a chain polymer of N-acetylneuraminc acid residues. The polysialic acid of the vaccine of the invention may be selected from Group B polysaccharide of *N. meningococcus* and K1 polysaccharide of *E. coli.*

The term adhesin molecule as used herein refers only to microbial or pathogen attachment molecules, whereas the term adhesion molecule refers to host attachment molecules.

### Diagnostic assay

A diagnostic assay in accordance with the invention comprises a monoclonal antibody specific for a microbial attachment molecule adhesin molecule that mimics host cell (adhesion) protein or carbohydrate molecules of a cell selected from the group consisting of leukocytes, endothelial cells, epithelial cells and other target cells of the host.

The monoclonal antibody adheres to the pathogen of interest. The monoclonal antibodies may be raised to the adhesion or attachment molecules of interest following the procedure set forth in Sambrook and Maniatis et al., *Molecular Cloning: A Laboratory Manual,* Cold Spring Harbor Press, 1989, incorporated herein by reference in its entirety.

The assay for detection, identification and enumeration of respiring microorganisms comprises the steps of:
a) passing a microbial sample through a collecting device to capture the cells;
b) adding to the collecting device a fluorochrome dye specific for the detection of respiring cells and allowing the dye to incubate;
c) treating the collected sample with a reactive fluorescent antibody which reacts with a microorganism of interest present in the microbial sample;
d) mounting said collecting device for examination by fluorescence microscopy in which a suitable light system is used to excite the fluorochrome dye and fluorescent antibody to fluoresce; and
e) quantifying, the fluorescence as a measure of the amount of said respiring target microbial cells.

Preferably the fluorochrome dye specific for the detection cf respiring microbe is taken up by respiring microorganisms and reduced to insoluble formazan crystals by the cytochrome system of said microbes. More preferably the fluorochrome dye specific for the detection of respiring cells is a tetrazolium compound. Most preferred is tetrazolium compound which is 5-cyano-2,3-ditolyl tetrazolium chloride (CTC).

In an alternative embodiment, the invention provides for a method for the detection, identification and enumeration of a respiring target bacterium comprising the steps of
a) mixing immunomagnetic beads comprising one or more antibodies which specifically bind to a target bacteria with a liquid sample to be tested;
b) allowing said liquid sample to interact with the beads for up to an hour;
c) placing the sample in a magnetic separator which causes the magnetic beads to which target bacteria have attached to separate from the liquid sample;
d) aspirating the liquid from the liquid sample, leaving the beads with bacteria attached;
e) washing the beads with a solution which removes loosely bound bacteria and other particles from the liquid sample;
f) mixing beads with bacteria attached with a fluorochrome dye specific For the detection of respiring bacteria;
g) treating bacteria on the beads with a fluorescent stain or a specific fluorescent conjugated antibody;
h) mounting said sample for examination by epifluorescent microscopy, in which a suitable light filter system is used to excite the fluorochrome dye and fluorochrome labeled antibody; and
i) quantifying to Fluorescence said respiring target bacteria.

The assay may be performed using immunomagnetic beads in accordance with the disclosures of U.S. application No. 08/245,262 filed May 18, 1994 or PCT/US95/05971, incorporated herein by reference in their entireties. In addition, provisional United States Patent application number 60/007,477, corresponding to this application is incorporated by reference in its entirety.

### Therapeutic Peptides

The invention also encompasses therapeutic peptides which comprise a molecule which mimics the adhesin molecule of a pathogen and interacts with receptor molecules of a host cell selected from the group consisting of leucocytes, endothelial cells, epithelial cells and other target cells of the host.

In a preferred embodiment, the therapeutic peptide molecule reacts specifically with receptor (ligands) of the selectin family of host adhesion molecules. The therapeutic peptide molecule preferably may react with receptors of the immunoglobulin superfamily of host adhesion molecules. The peptide molecule alternatively reacts with receptors of the integrin family of host adhesion molecules or mimics the host cell adhesion molecule and interacts with the adhesion molecules of the pathogen.

The peptide alternatively reacts with a receptor of the cytokine or chemokine family of host adhesion molecules or mimics the host adhesion molecule and interacts with adhesin molecules of the pathogen.

The inventors have found that naturally occurring endogenous peptide antibiotics of vertebrates act as an effective deterrent to viral, bacterial, fungal, and parasitic infections. Gene-encoded peptide antibiotics, are ubiquitous components of host defenses in mammals, birds, amphibia, insects, and plants. Their *de novo* synthesis or release from storage sites can be induced rapidly, which makes them particularly important in the initial phases of resistance to microbial invasion. The endogenous anti-microbial peptides of animals are usually products of single genes and are synthesized as preproteins. Multistep processing yields the mature peptide or group of peptides, which can act by inducing microbial membrane permeabilization or by adhesion to the microbial surface. The adhesive peptides act as lectins binding to the carbohydrates or lipids presented on the surface of infectious microbes thus making the organism more susceptible to immune detection and clearance. These binding peptides may also act to block the microbial host adhesion events leading to infection.

Several families of antimicrobial peptides have been identified that differ with respect to the presence of disulfide linkages, amino acid composition, structural conformation, and spectrum of activity. These peptides are remarkably abundant and widely distributed in animals and plants. The role of antibiotic peptides of higher eukaryotes, as therapeutic compounds, is a central theme in natural immunity. Type C lectins or carbohydrate binding lectins which characterize selectins as well as a host of microbial attachment molecules as listed in Microbial Lectins and Agglutinins, 1996, John Wiley and Sons, NY. This reference identifies nearly 60 microbial species that use lectins to bind to carbohydrate ligands on host target cells. Microbial lectins, like those on inflammatory cells of the host, serve as molecules of recognition in cell-cell interaction, for example, bacteria-epithelial or pragocytic cell interactions. In addition, they are involved in signal transduction events that prepare the host cell for entrance of the parasite, its intracellular growth and eventual death of the cell. Lectins bind reversibly and noncovalently with mono or oligosaccharides, both simple and complex, and whether free in solution or on cell surfaces. These cell surface sugars are referred to as lectin receptors or ligands and define the specificity of the lectin. Lectins frequently appear on the surface of the cell, on specific organelles, such as bacterial fimbriae, or are part of the structure of exotoxins elaborated by bacteria.

Naturally occurring, or engineered, mAbs or pepcides with anti-adhesive properties targeting host-parasite adhesion associated signaling and adhesion are effective for producing diagnostics, therapeutics and vaccines for host infections requiring microbial binding to host tissues.

### PATHOGENICITY DEFINED BY ADHESION EVENTS

Many pathogens trafficking through the vascular system use adhesion pathways operating under shear as a means of seeding various tissues. The inventors believe this concept is universally applicable and show the use of host adhesion pathways unique to different anatomical systems (respiratory, gastrointestinal tract, urinary tract, etc.) by a given pathogen. Thus, a new approach to defining the virulence of many bacterial, viral, fungal and parasitic organisms may be directed to the detection and characterization of various prokaryotic and eukaryotic adhesion proteins that infiltrate the host's communications network. Several studies on different host-parasite relationships reveal how pathogens use the cell-cell adhesion systems of the host during the course of an infection. The results of some salient studies are presented to illustrate the types of functional mimicry that has evolved to allow a microbe to infiltrate the host cell-cell networks and to direct the course of infection.

Many pathogens disseminate or traffic from the portal of entry to distant tissue and organs by entrance into and transport by the vascular system. In several cases, pathogenic bacteria, fungi, and protozoa have been shown to adroitly manipulate the host's adhesion system, including the selectins and integrins, during their pathogenesis.

### Molecular Basis for the Perversion of the Host Cell-Cell Communication Network by Pathogens

Although it is clear that protein-carbohydrate interactions control a myriad of biological events, the low binding constants for protein-carbohydrate couples have raised questions regarding the precise role of carbohydrate ligands in biological communication. However, a model for microbial toxin-host cell interaction proposed by St. Hilaire *et al.* (21) calls attention to the similarities of protein -carbohydrate interactions found in microbial adhesion systems to those employed by the host and notes that the latter interactions are involved in specificity but not tight binding of the receptor/ligand pairs. Tight binding appears to be promoted by protein-protein or protein-lipid interactions. This principle has been demonstrated for neutrophil recruitment where recognition is mediated by protein (selectin)-carbohydrate (sialyl Le) binding but tight binding and flattening are the result of protein-protein (integrin/ligand) or protein-lipid binding. Similarly, St. Hilaire et al. have shown that the adhesion of the B-subunit (one of the smallest known lectins) of the VTEC holotoxin invokes both protein-carboydrate (for targeting the toxin) and protein-lipid interactions. (for entry of the toxin into the cell) which are also reminiscent of the mode of action of botulism, pertussis and diphtheria toxins (22). The generality of this motif requires structural and energetic studies on a large array cf protein-carbohydrate-lipid-mediated biological recognition systems.

The recognition of, and the Tight binding to, target cells of the host by a pathogen or a toxin is a prelude to the activation of signaling pathways that produce alterations in the actin network of eucaryotic cells (23). Generally, these second order signal transductions trigger destructive events including increases in cytosolic free calcium, rapid rearrangement or disruption of the actin cytoskeleton and lysosome recruiment and clustering that often conclude with the death of the cell. Many pathogenic bacteria, largely gram-negative microorganisms, have acquired and apparently readily share genes encoding proteins elaborated by a specialized (type III) secretion system which is triggered by binding to the host cell (24). These novel virulence proteins are distinquished by the lack of a signal sequence that normally permits a secreted protein to pass through membranes but are presumed to contain attachment structures for specific proteins in the cytosol. They appear to gain entry as a consequence of a specific protein-carbohydrate interaction between pathogen and target cell followed by protein-protein tight binding that promotes the necessary structural and energetic changes for signal transduction within the cell

### Examples of Molecular and Functional Mimicry of Host Adhesion Systems by Microbial Pathogens

Several studies on different host-parasite relationships have begun to reveal how pathogens use the cell-cell adhesion systems of the host during the course of an infection. The results of some salient studies illustrate the types of functional mimicry that has evolved to allow a pathogen to infiltrate the host cell-cell networks and to direct the course of infection. Host and microbial adhesion molecules are highlighted in each system.

### THE LEUKOCYTE/ENDOTHELIAL CELL COMMUNICATION NETWORK

Adhesion to vascular endothelial cells is required for leukocytes to exit from the blood into tissues. These interactions are controlled by adhesion proteins expressed by both the leukocyte in the blood and the endothelial cell. The inventors have characterized the function of several adhesion proteins in the recently described family of adhesion molecules called selectins (4, 5, 6, 7) . Selecting are so termed because their lectin-like structures mediate selective adhesive interactions by recognition of carbohydrates on target cells (8). One leukocyte selectin (L-selectin) and two vascular selectins (E- and P-selectin) have been defined. In addition to endothelial cells, platelets also express P-selectin. L-selectin is constitutively expressed by leukocytes, whereas, stimulation of endothelial cells with immune cytokines, histamine or traumatic insult is required to induce E- and P-selectins. In addition to selectins, other adhesion proteins, such as VCAM-1 on cytokine-stimulated endothelial cells (9), MADCAM-1 on high endothelial venules of the gut, PNAd-1 on high endothelial venules in peripheral lymph nodes (10) , high carbohydrate-containing, mucin-like molecules (11,12), and some integrins (VLA4 and LPAM-1) (13,14) also mediate cell-cell interactions. Integrins are key adhesion proteins that work by triggering the endothelial or epithelial cell to loosen tight junctions in preparation for leukocyte or microbial transmigration.

In most instances, adhesive interactions between leukocytes and endothelial cells are represented by rolling of the leukocyte along the endothelial monolayer. These rolling interactions can cause >1000-fold reductions in the velocity of the leukocyte moving through the vascular system. Analyses of leukocyte/ endothelial cell interactions demonstrate that specific classes of adhesion proteins preferentially mediate binding under shear forces characteristically found in the vascular system (15,16,17,18.19). For example, many well characterized static adhesive interactions between leukocytes and endothelial cells [for example, those mediated by the beta-2 integrins (LFA-1, MAC-1)] were found not to take place in *in vitro* assays under high shear conditions designed to reflect blood flow (17,19). In contrast, adhesion molecules have been identified which preferentially function under shear. Table 1 provides a list of defined leukocyte-endothelial cell adhesion proteins involved in extravasation and indicates whether they function best under shear or static conditions.

**Table 1**

| Adhesion Molecules Examined Under Shear Adhesion Molecules Expressed on Optimal Function Conditions | | | | |
|---|---|---|---|---|
| | Leukocyte | Endothelium | Shear | Static |
| E-selectin | - | + | + | - |
| P-selectin | - | + | + | - |
| L-selectin | + | - | + | - |
| MAdCAM-1 | - | + | + | + |
| PNAd - | + | + | +/- | |
| VCAM:-1 | + | + | -/+ | + |
| ICAM-1 | + | + | - | + |
| Mac-1 | + | - | - | + |
| 1-LFA-1 | + | - | - | + |
| VLA4 | + | - | -/+ | + |
| beta -1 | + | + | -/+ | + |
| LPAM | + | - | + | + |

The inventors conclude that the rolling of leukocytes along the vascular endothelium allows other adhesive interactions to take place which cement leukocytes to the vascular endothelium. Migration or trafficking through the vascular lining then takes place. Thus, three steps are involved in successful arrest of leukocytes along the vessel wall:
1) shear-dependent attachment and rolling involving selectins.
2) activation dependent adhesion strengthening (slowed rolling), followed by tight adhesion
3) transendothelial cell migration governed by inducible integrins on the endothelium.

All three steps are required for the effective recruitment of inflammatory leukocytes to sites of injury or infection, or the seeding of lymphoid tissues.

The isolation of microbial mimics of immunoglobulin superfamily molecules, ICAM1-3, VCAM, NCAM and PECAM, is accomplished as described herein. The interactions of suspected IG superfamily mimics with known ligands, integrins for example, is part of the characterization of the microbial mimic.

There are numerous examples of both prokaryotic and eukaryotic adhesive proteins some of them being glycoproteins as described in earlier responses. All of the carbohydrate binding lectins including the selections and microbial lectins are largely glycoprotein in nature and are decorated with carbohydrates in varying degree.

Eukaryotic adhesive proteins include the selectins, integrins, and Immunoglobulin superfamily and an array of adhesive molecules that promote cell-cell signaling and recognition such as CD4 and CD8 on T cells, GTP-binding proteins (RhoA) , cytokines and growth factors (IL-n, TGF-β1 and the hepatocyte growth factor). More than five chemokine receptors have been identified in leukocytes and appear to play a major role in the trafficking of leukocytes.

A major effort in host parasite interactions now focuses on the signaling events initiated by adhesive microbial cellular and secreted compounds that promote changes in the actin network of host cells such as host leukocytes, epithelial cells, endothelial cells and nervous system cells.

Leukocytes that traffic in the blood are variously classed as a) lymphocytes, b) neutrophils, c) monocytes and d) platelets . Other cells that interact with or are derived from blood borne cells include antigen presenting cells (M Cells, dendritic cells, macrophages) and cells that elaborate or secrete pharmacologically active compounds (mast cells, eosinophils). The lymphocyte population includes subsets of antigen-reactive T cells defined by different functional roles they play (cytotoxic, helper cell, suppressor etc.) and the combination of surface antigens or receptors they display. Two distinct subsets of T cells are instrumental in directing the immune response of the host into cell-mediated immune response (Th1) or predominantly a humoral immune response (Th2). Another lymphocyte population, the B cells, is characterized by the presence of membrane-bound immunoglobulins, initially monomers of IgM and later, IgD molecules and, after maturing into plasma cells, produce and secrete immunoglobulins.

Macrophages develop from blood-borne monocytes and bear a number of cell surface receptors for antibody, complement and various cell-cell interactions chat promote movement through endothelia and epithelia. Dendritic cells are also large, motile cells and, like macrophages, are involved in the processing/presentation of antigens to lymphocytes.

The cells of the immune system and many of those involved in the inflammatory cell response circulate as nonadherenc cells in the blood and lymph and migrace as adherent cells through tissues. In the presence of a foreign antigen or inflammatory stimulus, they must be able to congregate in lymphoid organs, cross endothelial and basement membrane barriers to aggregate at sites of infection, and adhere to cells bearing foreign antigen. There is a rapid transition between adherent and nonadherent status of leukocytes that is key to the dual functions of immune surveillance and responsiveness. The three major families of adhesion receptors direct the localization and migration or homing of leukocytes to different tissues of the host. The Immunoglobulin superfamily is found in the antigen-specific receptors of T and B cells, the integrin family is important in the dynamic regulation of adhesion and migration of leukocytes across epithelia and endothelia, and the selectins are prominent in leukocyte interaction with vascular endothelium. In some cases, counter receptors or addressins for these adhesion molecules have been found to be tissue or cell specific which causes leukocytes to home to Peyer's patch, skin or kidney endothelium. Platelets are intimately associated with changes in vascular physiology through their interaction with leukocytes and endothelial cells and the elaboration of pharmacologically active agents that are associated with infections and inflammation. P-selectin is stored in α granules of platelets and Weibel-Palade bodies of endothelial cells and is rapidly mobilized to the surface of these cells after stimulation by products of the clotting cascade such as thrombin where it mediates adhesion of neutrophils and monocytes. These factors are operative in reperfusion and traumatic injury and other vascular events that impair the function of tissues and organs.

Epithelial cells line the lumenal surfaces of the intestinal tract, genito-urinary tract, upper respiratory tract and various organs of the mammal. Epithelial cells express many members of the integrin family and possess a host of different glycoconjugates that function as counter-receptors for pathogen adhesion molecules (PAMs).

Examples of nervous system cells include astrocytes, glial cells, Schwann cells and neurons. The dominant host adhesion molecules found on nervous system cells are NCAM and ICAM. Ligand counter-receptors that bind to PAM are sialic acid-rich structures.

Endothelial cells may display one or more adhesion molecules depending on (a) stimulation by inflammatory mediators and (b) their location in tissue or an organ environment. The ICAM subfamily is generally expressed on endothelial cells as a consequence of stimulation by inflammatory mediators including lipopolysaccharide, interferon, interleukin-1 and tumor necrosis factor (TNF) elaborated during infections or tissue damage. VCAM has induction kinetics and function similar to ICAMs but interacts only with the VLA-4 integrin while ICAMs bind to LFA-1 integrin. Examples of dominant host adhesion molecules on endothelial cells include ICAM-1, VCAM-1, MAdCAM-1 (Peyer's Patch), and PNAd-1 (limph node).

Lymphocytes in the blood enter lymph nodes by binding to specialized "high" endothelial, cells. Molecules, termed "addressins" selectively expressed on specialized HEV in different types of lymph nodes function as homing receptor ligands. Thus, endothelial cells expressing MAdCAM-1 are found in the high endothelial venules of the Peyer's patches and lamina propria, and the lymphoid system associated with the intestine. Endothelial cells expressing PNAd-1 are found in peripheral lymph nodes.

The isolation of a pathogen's attachment molecule which mimic those expressed on endothelial cells begins with its detection and characterization in the inventors *in vitro* or *in vivo* adhesion assay systems using target cells that express the ligand for the attachment molecule. For example, to detect a MAdCAM-1 like molecule used by the pathogen, a target cell system expressing the MAdCAM-1 ligand, the α4β7 integrin would be used. Fibroblast transfectants expressing the integrin could also be employed in the assay. If adhesion is detected the specificity is verified by treatment with anti-MAdCAM-1 monoclonal antibodies (MECA-367) to block the adhesion of microbe to the target cell (Bargatze, R. et al. 1995 Immunity. 3:99-108). Similarly, VCAM-like or ICAM-like attachment molecules would be detected in assays using target cells displaying the integrins α4β1 or LFA-1, respectively.

Whooping Cough - *Bordetella pertussis* uses its **filamentous hemagglutinin** (FHA) to interact directly with a leukocyte integrin signal transduction complex to move into and out of the vascular network (25) The signature of the molecule which binds to integrins is the Arg-Gly-Asp triplet. This sequence has been found in over a dozen other prokaryotic proteins, often as part of an adhesive domain. Since integrins play a critical role in joining neighboring cells in most mammalian cell systems, certain pathogens have learned to use this recognition system for passage across endothelia and epithelia. Through this mechanism, B. pertussis enhances its own attachment and potential for systemic dissemination through co-opting a host cell adhesion signaling pathway. The FHA has been demonstrated to be a key colonizing factor in mouse respiratory infections. Its role in infection has been successfully blunted by blocking-with anti-FHA mAbs (26).

Malaria - Erythrocytes infected with *Plasmodium berghei* and *P. falciparum* are responsible for the frequently severe clinical complications associated with cerebral malaria. These complications are a direct result of the infected erythrocytes obstructing blood flow in the brain by adhering mechanically to themselves and the endothelium. In a study examining the role of vascular and leukocyte adhesion molecules in a mouse malaria model, it was shown that erythrocytes infected with F. berghei were prevented from binding to brain endothelium and occluding blood flow by intravenous treatment with mAb directed against the LFA-1 (an integrin) adhesion molecule (27). Another study examining erythrccytes from human patients infected with *P. falciparum* demonstrated. specific adhesion to E-selectin and VCAM-1 as a means of attachment to human brain endothelium (28).

Candidiasis - Research on *C. albicans* has shown that it expresses an phosphomannoprotein on its surface responsible for the ability of hematogenous yeast cells to adhere (presumably to an integrin) on mouse splenic marginal zone macrophages and peripheral lymph nodes (29). *C. albicans* binding to fibronectin in the extracellular matrix of blood vessels has been implicated as playing a direct role in promoting fungal adhesion for blood disseminated fungal disease (30).

### Respiratory Tract Pathogens

The respiratory tract presents three environments to pathogens: the ciliated epithelium of the nasopharnyx, the alveoli and the alveolar macrophage. Pathogens attaching to the ciliary epithelium rely on carbohydrate addresses for tissue tropism. The localization of classical pathogens of pneumonia to the alveolus is also based on carbohydrate recognition, but the molecular participants are not known. The most definitive work that reveals the functional similarity of a microbe's address recognition and signaling system with that of the host's involves the alveolar macrophage system (26).

Macrophages enter the lung by employing the CD18 family of adhesion molecules, LFA-1, Mac-1 and gp.150.954, which recognize ligands on inflamed endothelia. Another less frequently used pathway involves a non-CD18 pathway provoked by pneumococcus and involves a platelet-activating factor as a key mediator. Pathogens which enter and reside within the alveolar macrophage use the CD18 family of adhesion molecules as the means of entering macrophages and circumventing the otherwise lethal oxidative burst from the leukocyte. Legionellas, mycobacteria, streptococci and histoplasmas bind to a variety of CD11/CD18 determinants, either on the integrin itself or on natural bridging ligands such as C3bi (1).

The most definitive studies on the role of microbial adhesion and signaling in the infectious process have been conducted on infections with Bordetella pertussis (35,32). *B. pertussis* presents two adhesion ligands to macrophages: pertussis toxin (PT) and filamentous hemagglutinin (FHA) described above. PT is a hexamer, the B oligomer of which is also a lectin with carbohydrate preferences similar to C-type selectins: the fucosylated polylaciosamines. The lectin-like feature of PT directs the adherence of *B. percussis* to cilia and macrophages. The cell recognition properties of PT have been localized to two protein subunits, S2 and S3, that are 80 % homologous and are identical to a region of residues (15 to 55) found in type C selectins. This sequence similarity extends to shared function in that the subunits can inhibit the interaction of neutrophils with selectin-coated surfaces.

A second property of PT is the ability to upregulate the function of the integrin family of leukocyte adhesion molecules. The ligation of a carbohydrate by the PT adhesion event upregulates the integrin CR3 (Mac-1) which allows the second adhesin FHA, a specific ligand for CR3, to adhere and trigger uptake and survival of the bacteria in the macrophage. Thus, the PT adhesion has two functions in the infectious process: address recognition and signaling the macrophage to increase receptor sites for the organism.

Other diseases in which the pathogen appears to have "learned" to use alvealor macrophage adhesion systems to initiate an infectious process and, in many cases, exploit their ability to use host adhesion molecules to spread to other tissues and organs include disseminated histoplasmosis, pulmonary blastomycosis, nocardiosis, cryptococcoses, bronchopulmonary candidiasis, pulmonary aspergillosis, Legionnaires disease and tuberculosis.

### Gastro-intestinal Tract Pathogens

The gastro-intestinal tract presents several environments to the pathogen beginning with the acidic environment bathing columnar epithelium of the stomach to the brush-bordered epithelium of the duodenum, jejunum, and ileum and the cuboidal epithelium of the colon. The lumenal face of the gastro-intestinal tract is coated with mucin produced largely by highly specialized goblet cells embedded in the epithelial cell layer. Mucin differs in chemical composition frcm one region to the next and even from cell to cell within regions of the intestinal tract. Glycoproteins rich in sialic acid residues, mucins appear to play an important role in protecting the underlying epithelium from deleterious effects of microbial activity.

The normal bacterial flora of the gut plays an important role in directing the course of infections of the GI tract. The sheer mass of organisms in the bowel (ca. 10¹⁰ per gram) , the variety of species present which promotes competition and antagonistic relationships and the genetic interactions between them creates an exceedingly complex relationship with the host. Pathogens of the gastrointestinal tract include a large array of parasitic species of bacteria and fungi.

Intestinal bacterial pathogens referred to collectively as enteric bacilli are formally classified under several genera that share common antigens and are genetically interrelated. Among the shared properties of enteric bacilli are factors contributing to their virulence including adhesion molecules, exotcxins and endotoxins. As a consequence of exchanging new genetic factors between enteric organisms, new strains arise to add to the already large array of intestinal pathogens. A case in point is the Escherichia coli pathotype 0157 :H7 which belongs to a recently evolved pathogenic clone possessing the secondary virulence factors, the Shiga-like cytotoxins (verotoxins) and plasmid-encoded adhesions (34).

The histopathology of many enteric bacterial infections, attachment and effacing (A/E), is a product of specific adherence of the pathogen to intestinal epithelia and the participation of endotoxins and, in some cases, potent exotoxins (35) . Most enteric pathogens demonstrate A/E forms of bacterial binding both to tissue culture cells *in vitro* and to enterocytes and colonocytes *in vivo.* The mediators of cell adhesion appear to contribute to a complex phenotype dependent on a gene cluster (eaeA loci) present on the bacterial chromosome and/or a plasmid-encoded factor designated EAF (EPEC adherence factor) (36,37). Many serotypes of verocytotoxic *E. coli* (VTEC) , encerotoxigenic *E. coli* (ETEC) and enteropathogenic *E. coli* (EPEC), *Hafnia alvei and Citrobacter fruendi* are eaeA positive and possess similar adhesion phenotypes. The A/E lesions produced by eaeA-negative *E. coli* (38) and the gastric pathogen *Heliobacter pylori* (39) lack detectable recruitment of underlying cytoskeletal elements.

Two forms of adherence derive from the expression of eaeA genes and/or plasmid encoded loci (40,41,42). The first or initial (localized) adherence is promoted by an 19 kDa EAF fimbrial adhesin referred to as the bundle forming pilus (BFF). The BFP adhesion causes adherence to the surface of the microvilli and the initiation of events that lead to effacing. Interestingly, the amino acid sequence of BFP of *E. coli* is similar to the pili of *Vibrio cholerae, Neisseria gonorrhoeae, Neisseria meningitidis*, and *Pseudomonas aeuroginosa,* all of which are members of the type IV fimbrial family. It is proposed that the 19 kDa adhesin protein reacts with a carbohydrate ligand on the epithelial cell or, less likely, a specific carbohydrate epitope associated with mucin. This adherence and subsequent microcolonization event is followed by the transduction of a signal that results in elevated intracellular calcium levels, activation of host cell protein tyrosine kinases, fluid secretion and in severe verotoxic *E. coli* infections, profound changes in cytoskeletal structures. A second adherence event promoted by a chromosome-encoded 94-kDa outer membrane protein (OMP) containing an adhesion structure referred to as intimin permits the organism to adhere intimately to the epithelial cell plasma membrane and appears to amplify cell signaling leading to marked effects on cytoskeletal proteins of the epithelium and, in some instances, to invasion of the submucosa. The amino acid sequence of *E. coli* intimin is highly similar to those of the invasins of *Yersinia psuedotuberculosis and Y. enterocolitica* (44). Invasins bind with a high affinity to members cf the beta-1 family integrin receptors to mediate the efficient uptake of bacteria. There is reason to believe, therefore, that OMP adhesions are used by pathogenic *E. coli* to pass through beta 1 integrin doors (in contrast to beta 2 integrins in respiratory infections) and maneuver along the cytoskeleton of epithelial cells.

*Helicobacter pylori* has effectively colonized the majority of the world's population and is now accepted as the major cause of chronic gastritis and the formation of peptic ulceration (45). The bacterium is tropic for epithelial cells and the mucus layer in the stomach lining. Adherence to gastric epithelial cells is mediated by lectin-like adhesins interacting with fucosylated blood-group antigens associated with blood-group O phenotype (46). Surface epithelial degeneration is a probable result of the activity of bacterial products including cyotoxins, urease and pro-inflammatory products such as LPS.

Some serotypes of *Escherichia coli*, *Shigella dysenteriae* (Type 1) , *Salmonella typhimurium* and *Vibrio* cholerae produce a potent 71 kDa multi-peptide subunit cytotoxin (Shiga like or verotoxins) comprised of a single 32 kDa "A" subunit and 5 x 7.7 kDa "B" subunits (22) . The B-subunits facilitate binding of the holotoxin to high affinity (Kd = 10¹⁰ M) cell surface receptors on and entry into the endothelial cells of the host. The endothelial cell receptor is a glycolipid, galactose ∂-**1,4-galactose-ß-1,4, -glucose ceramide** (Gb3) (47). The "A"-subunit inhibits protein synthesis in the endothelial cell at the riboscmal level.

Microbial adherence as a mediator of infections by *E. coli and H. pylori* is more than likely descriptive of infections caused by *Shigella species, Vibrio cholerae* and *Salmonella species* as well as many protozoan parasites (*Entamoeba hiscolyicica)* and viruses. In positioning the pathogen on the epithelial surface, the stage is set for the acticn of other virulence factors (toxins, enzymes, etc) that alter or efface the epithelial surface. These adhesive interactions may also serve to isolate the pathogen from the antagonistic behavior of the normal flora and the flushing action of the peristaltic movement of the gut.

### The Central Nervous System

Bacterial meningitis appears to be the consequence of trafficking of microbes from an initial site of infection to cells of the meninges. In the case of infection with Neisseria meningritidis, the portal of entry is the nasopharynx, where organisms may persist for long periods of time without overt signs of infection. Meningitis caused by E, coli, on the other hand, has its origin in the gastro-intestinal tract or, less frequently, the lower respiratory tract and the urinary tract.

Bacterial adherence most likely plays a role in establishing infection at the portal of entry which involve the recognition of specific carbohydrate receptors on epithelial cells.

The studies by Finne (48) support the notion that polysialic acid, as part of a glycoprotein carbohydrate, plays a key role in recognition of cellular addresses in the brain. The polysialic acid chain is a polymer of N-acetylneuraminic acid residues joined by alpha 2-8 linkages. There are as many as 12 or more carbohydrate residues per chain. Sialic acid is bound to a core glycan which seems to be of the N-linked tri- and tetra-antennary type commonly encountered in glycoproteins. The brains of young mammalians are highly sialylated in contrast to less sialylation and shorter chains in adult brains. Various studies suggest that the major role of polysialic acid is to modulate cell adhesion activity in the brain which suggests that a specific molecular address for trafficking microbes bearing sialic rich capsules exists in the adhesion molecule of meningeal cells.

The capsular type-specific polysaccharides of meningococci are polymers of acidic sugar residues rich in sialic acid. Most are effective immunogens but the group B polysaccharide of meningococci and the K1 polysaccharide of *E. coli* stimulates low titers of low affinity, IgM class antibody. The poor response to B and K1 polysaccharides is most likely the consequence of immunological unresponsiveness co capsular material that is chemically identical to brain polysialic acid. In any event, the recognition of an adhesion receptor in brain cells by meningccocci or K1 bearing E. coli provides an explanation for the tissue tropism of these organisms.

### Other Microbial Adherence Systems

*Neisseria gonorrhoeae* is an obligate pathogen of man that initiates infection of the genitourinary tract by adherence to epithelial cell surfaces (40). This attachment is mediated by pill-associated adhesion since only piliated strains are able to adhere to the cells. Upon attachment, the gonccocci appear to become engulfed by epithelial cells following some form of signaling event by an outer membrane protein (50). Many gonococci penetrate between columnar epithelial cells to reach subepithelial tissues presumably by activating an integrin receptor associated with epithelial cell junctions.

Leishmaniasis includes a group of diseases caused by one of several species of protozoa belonging to the genus Leishmania. All of these protozoa are transmitted by the bite of a small sand fly of the genus Phlebotomus. The major surface glycoprotein of Leishmania, gp63, a fibronectin-like molecule, plays a key role in parasite-macrophage interactions (51). The subsequent growth and persistence of the parasite in macrophages contributes to the extensive involvement of some or all of the reticulo-endothelial system during the disease.

Herpesvirus. Of all known viral proteins, few have signif icant sequence similarity to host cellular proteins but viral agents rely on recognition molecles on the host cell for attachment and entry. However, the RNA retroviruses and two classes of DNA viruses x, the poxviruses and herpesviruses, employ molecular mimicry of cytokines to subvert host cellular functions (20). The herpesvirus chemckine-receptor homologs mimic host membrane proteins transmitting a signal to the cytoplasm cf host leukocytes that ensures a cytosolic milieu for herpesvirus replication and/or the establishment of latency.

The revolutionary conclusion the inventors have drawn from these studies is that most, if not all, pathogens have "learned" to infiltrate the host's network by using adhesion molecules that functionally mimic those of the host. The applications of the present inventors understanding of the functional and molecular character of prokaryotic adhesion molecules has led to the development of a new generation of diagnostic reagents, therapeutic compounds and vaccines.

The inventors have developed new reagents to detect potent immunogens for immunization against functionally similar but antigenically distinguishable adhesion molecules both of microbial origin and identified through "in vitro evolution". In addition new adjuvants are developed that recruit a specific population of immunologically competent cells into organ and tissue sites (i.e., mucous membrane of the intestinal tract or nascpharyngeal region) where infectious adhesion events are likely to take place.

The invention abrogates microbial-host signaling and adhesion and is a novel approach for the development of diagnostics, therapeutics and vaccines. The similarity of leukocyte and microbial trafficking events, most notably address recognition, signal transduction, and passage through endothelia and epithelia, implies that many microbial adhesion and signaling systems are evolutionary products of functional, as well as molecular, mimicry of host protein and carbohydrate moieties . This convergence of cell biology and microbial pathogenesis predicts that for each system of mammalian cell trafficking there exists a pathogen that is intimately familiar with the network.

The function and characteristics of several pathogen-host cell adhesion systems have been identified by the inventors using an *in vitro* shear assay system. These host-parasite adhesion systems include but are not limited to verocytotoxic *E.* coli, *T. foetus, C. albicans*, and Hanta virus . Monoclonal antibodies have been developed against microbial adhesion factors to (1) aid in the detection and characterization of adhesions and their counter receptors on target cells and (2) define the dynamics of in vitro adherence events with appropriate target cells. The invention defines the nacure and function of adhesion interactions in each in vitro target cell-pathogen system.

In one embodiment, the invention allows for the:
1. Preparation of aggregates or suspensions of pathogens for use in adhesion assays.
2. Preparation of primary cultures of target cells for adhesion assays.
3. Assessment of the adhesivity of pathogens with target cell receptors under shear.
4. Evaluation of the affinity of adhesive interactions of pathogen with target cells under different levels of shear force.
5. Testing of the effect of anti-adhesion reagents including anti-selectins and anti-integrins on the target cell-pathogen interaction.

There are several examples of functional equivalents (host homologs) to integrin molecules. The integrin-like region on 1) the surface of Rhinovirus that binds to ICAM-1 endothelial cells; 2) the surface of *Candida albicans* that binds to ICAM-1; and 3) the surface of *Entameba hiscolytica* that binds to N-acetyl-galactosamine ligands on intestinal epithelium.

To prepare and use in vaccine formulations, the integrin-like attachment molecule (adhesin) is defined and prepared as described herein. The key to success is dependent on (1) an amino acid sequence of the adhesive site of the adhesin that is encoded by microbial genes, hence is foreign (immunogenic) to the mammal and (2) presentation to the immune apparatus is in an aggregated form or a vehicle that stabilizes and protects it from degradation *in vivo*.

The integrins VLA, Leucam and cytoadhesions, in their native and functional form, are not used in a vaccine preparation because they are of host origin. Instead, microbial peptide homologs of the host's integrin molecules that react with similar ligands on host cells or extracellular matrix proteins are used. Alternatively, or in conjunction thereto, molecules developed via *"in vitro* evolution", such as through phage-display selection, may be used. In the thesis, the parasite or pathogen has acquired, by chance or, more likely, genetic piracy, a glycoprotein, glycclipid or carbohydrate structure that is functionally similar but antigenically distinct from the host receptor molecule (integrins, selectins or carbohydrate, protein or lipid ligand structures).

Identification of the adhesion associated signaling and adhesive receptors and ligands for both the pathogen and the host provides for the development of a vaccine comprising an adhesin molecule of a pathogenic organism (or functional analog) which interacts with receptor molecules of a cell selected from the group consisting of leukocytes, endothelial cells and epithelial cells or an adhesion molecule of a host cell (s) (or functional analog) which interacts with adhesion molecules of a pathogenic organism.

### A New Generation of Biologics: Adhesion blockers

The demonstration of effective blocking of adhesion events involved in well defined infectious disease models has provided the basis for developing new vaccines, therapeutics, and diagnostics for many bacterial, viral, fungal, and protozoan infections. This "Adhesicn Blocking Technology" (ABT) generates a series of new products and applications for use in both humans and animals. A few examples of pathogens whose trafficking in the host is a function of adhesion-mediated events and for which effective new and novel diagnostics, therapeutics, and vaccines consisting of or containing adhesion-based reagents are developed include, for example, *Salmonella, Shigella, vibrio, Yersinia, Bordecella*, *Legionellas, Candida, Helicobacter, Neisseria, Histoplasma, Leishmania species,* Influenza, Hantavirus, HIV virus, *Escherichia coli (VTEC)*, *Trichomonas, Eimeria, Trypanosoma, Epstein-Barr virus, Pneumococcus, Streptococcus, Rubella,* Herpes, *Staphyloceccus, Chlamydia and Giardia.*

### Benefits from use of "Adhesion Blockers"

"Adhesion blocker" technology, forms the basis for new generations of vaccines, therapeutics, and diagnostics and offers the following advantages over existing therapeutic and detection technologies.
- Vaccines -Development of a new, highly specific and tissue-targeted subunit vaccine technology will provide a more effective and less costly family of vaccines that can be delivered orally or nasally eliminating the need for invasive injections. The inventors develop reconbinant vaccines such as that described by Wu *et al.* (54) consisting of live vector delivery systems displaying critical peptide domains of microbial (adhesive) antigens that target the adhesive antigens to inductive sites of specific cellular (e.g. immune) compartments. The resulting immune response has been shown to be effective at both the mucosal surfaces and in nearly all of the systemic immune compartments (55).
- Therapeutics - The utility of a new generation of therapeutic compounds has been demonstrated in studies that show adhesive peptides (56) and carbohydrates (57) can be used to block cell-cell adhesive interactions. Anti-microbial thereapeutics including both peptide mimics of adhesive receptors or carbohydrates are, therefore, useful for treating and preventing the pathogen from attaching to receptor ligands of host cells. The inventors define the amino acid sequence of adhesive peptide domains using anti-adhesion monoclonal antibodies to probe a phage peptide display library (58) Such therapeutics are non-toxic, relatively in-expensive to prepare and administer and possess therapeutic and/or curative activity that is not thwarted by the development of "resistance" by mutations within the pathogenic species as is the case with antibiotic treatment.
- Diagnostics - The new generation diagnostics can detect and identify the major, common virulence factor (adhesion molecules) shared by most if not all subtypes of a given group of pathogen (e.g., *Salmonella species, Streptococci, etc.)* and, because adhesion molecules are most likely shed by most pathogens into blood and other body fluids, can be detected by appropriate antibody-based assays well before the onset of symptoms. Diagnostic assays under the invention are easy to use and inexpensive, such as "dip stick" technologies, some of which may be marketed as over the counter (OTC) products.

### EXAMPLE 1

The T-cell and antibody responses to a cell surface streptococcal antigen (SA I/II) are investigated in naturally sensitized humans as set forth in "T-cell, adhesion, and ß-cell epitopes of the cell surface *Streptococcus* mutans protein antigen I/II." Kelly, C.G., Todryk, S., Kendal, H.L., Munro, G.H., Lehner, T., Department of Immunology, United Medical School at Guy's Hospital, London, United Kingdom, Infect. Immun. (United States), Sep. 1995, Vol. 63, No. 9, pp. 3649-58.

Serum antibody responses are directed predominantly to the N-terminal residues 39 to 481) and central (residues 816 to 1213) regions of SA I/II which are involved in bacterial adhesion to salivary receptors . T-cell responses were also directed predominantly towards the central region. The linear peptide relationship of the immunodominant and minor T-and ß-cell as well as adhesion epitopes is mapped within residues 816 to 1213. Immunodominant T-cell and ß-cell epitopes are identified within residues 803 to 853, which are separated in linear sequence from the adhesion epitopes (residues 1005 to 1044). Adhesion epitopes overlap with minor ß- and T-cell epitopes (residues 1005 to 1054 and 1085 to 1134). An immunodominant promiscuous T-cell epitope (residues 985 to 1004) is adjacent to an adhesion epitope (residues 1005 to 1024). The limited ß-cell response to adhesion epitopes is consistent with the success of *Streptococcus* mutans in colonizing the oral cavity. The strategy of T-cell, adhesion, and ß-cell epitope mapping reveals a general approach for identifying components of subunit vaccines which may focus responses to critical functional determinants.

Epitopes of SA I/II constitute the components of a subunit vaccine against dental caries.

### EXAMPLE 2

Antisera are generated against each of seven synthetic peptides corresponding to constant and variable sequences of the pili from gonococcal strain MS11 and were assayed for their ability to crossreact with intact pili from both homologous and heterologous strains as in "Antibodies to peptides corresponding to a conserved sequence of gonococcal pilins block bacterial adhesion." Rothbard, J.B., Fernandez, R., Wang, L., Teng, N.N., Schoolnik, G.K., Proc. Natl. Acad. Sci. USA, Feb. 1985, Vol. 82, No. 3, pp. 915-9. The peptides elicit roughly equal antipeptide responses but vary substantially in their ability co elicit antisera that cross reacted with intact pili. Of the antisera to peptides corresponding to regions of conserved sequence, antisera directed against residues 69-84 are the most efficient in binding pili from all strains tested in both solid-phase assays and immunoblots. Anti-69-84 also efficiently precipitate a tryptic fragment of pili known to bind human endocervical cells. Sera against the two peptides (121-134 and 135-151) previously shown to contain strain-specific epitopes crossreact with MS11 pili equally well, but differ in their ability to bind pili from heterologous strains. Anti-121-134 is strain-specific whereas anti-135-151 bind all pili tested. Each of the sera is examined for its ability to inhibit bacterial adhesion to a human endometrial carcinoma cell line. Sera generated against residues 41-50 and 69-84 successfully inhibit a heterologous gonococcal strain from binding.

These peptides are components of an effective vaccine for the prevention of gonorrhea.

A diagnostic reagent or assay in accordance with the invention comprises a monoclonal antibody or peptide construct specific for an adhesin molecule of a pathogenic organism which interacts with a cell selected from the group consisting of leukocytes, endothelial cells, epithelial cells, and other targeted host cells.

### EXAMPLE 3

A genomic library of *Mycoplasma pneumoniae* is constructed by cloning sheared genomic DNA into the expression vector lambda gt11 as in "Identification of P1 gene domain containing epitope(s) mediating Mycoplasma pneumoniae cytoadherence." Dallo, S.F., Su, C.J., Horton, J.R., Baseman, J.B. Department of Microbiology, University of Texas Health Science Center, San Antonio, J. Exp. Med. (United States), Feb. 1, 1988, Vol. 167, No. 2, pp. 718-23.

Recombinant clones are screened using anti-M. pneumoniae mAbs reactive with adhesion P1 epitopes that mediate cytoadherence. Ten clones with different size inserts are isolated. These clones possess P1 sequences localized to the COOH terminus of the P1 gene. All clones produce fusion proteins that react with acute and convalescent sera of patients infected with M. *pneumoniae*. Interestingly, one clone, P1-7, contains an epitope that was confined to a region of 13 amino acids present in the *M. pneumoniae* genome. as a single copy.

The identification of this cytoadherence-related epitope permits the production of a synthetic peptide that can be used as a vaccine and serodiagnostic probe.

### EXAMPLE 4

A therapeutic peptide (or mAb or mAb fragment) comprises a molecule which mimics the adhesin molecule x of a pathogen and interacts with receptor molecules of a cell selected from the group consisting of leucocytes, endothelial cells, epithelial cells and other target cells of the host or a therapeutic peptide (or mAb or mAb fragment) comprising a molecule which mimics the host adhesion signaling or adhesion molecules and interacts with the adhesin molecules of a pathogen. The development of mAb probes to detect and define the peptide domains in and flanking the adhesive site of the attachment molecule is described herein.

Research into immunity to complex intracellular parasites has recently placed emphasis on the identification of peptide sequences recognized by T-cells, often with the dual objectives of finding species-specific protective epitopes, and of understanding selection of Th1 versus Th2 response patterns, see "Adjuvants, endocrines and conserved epitopes; factors to consider when designing therapeutic vaccines". Rook, G.A., Stanford, J.L., Medical Microbiology, UCL Medical School, London, U.K., Int. J. Immunopharmacol. (England), Feb. 1995, Vol. 17, No. 2, pp. 91-102.

However, the present inventors show the balance of Th1 to Th2 lymphocyte activity is not determined by epitopes, but rather by adjuvant effects of microbial components, and local endocrine effects mediated by conversion of prohormones into active metabolites by enzymes in lymph node macrophages. Cytokines play a role as mediators within these pathways. In chronic disease states there is a tendency for T-cell function to shift towards Th2. The inventors describe immunopathological consequences of this tendency, including a putative role for agalactosyl IgG, suggest the involvement of changes in the endocrine system, brought about not only by the cytokine-hypothalamus-pituitary-adrenal axis, but also by direct actions on peripheral endocrine organs of excess levels of cytokines such as TNF alpha, TGF beta and IL-6 . The inventors show that the epitopes that are targets for protective cell-mediated responses to complex organisms are usually not species specific.

In tuberculosis, cellular responses to species-specific components appear to be associated with immunopathology rather than protection. The application of the invention principles leads to remarkable results in the immunotherapy of tuberculosis, including multidrug-resistant disease.

### EXAMPLE 5

### Production of Monoclonal Antibodies

Monoclonal antibodies are produced in accord with well established techniques reported in prior publications (10,11). Hybridomas have been prepared from mouse spleen-derived B-cells immunized with either adhesion positive *Escherichia coli* or purified adhesion material prepared from extracts of *Candida albicans.* In all cases, Balb/c mice, ranging in age from 8 to 12 weeks, were used for hybridoma and antibody production.

Immunization protocol : Primary immunization antigens were adjusted to 10-20 µg of adhesion vaccines suspended in 0.5ml PBS and emulsified in CytRx® TiterMax® #R-1 at a 50/50 ratio. In all cases, the subcutaneous (SC) route was used to avoid a toxic reaction in mice. Immunized mice were bled at day 14 to test antibody responses by ELISA. If a low titer was found, the mice were boosted with similar dose of antigen and bled and tested at day 28. This procedure was repeated at 2 week intervals until the desired antibody titers for both antigens were achieved.

ELISA screening protocol: ELISA assays employed Costar® universal covalent surface immuno assay plates and standard protocols for binding of the bacterial antigens. The system employed a two or three stage development syscem in which the adhesin or verotoxin was covalently bound to the 96 well assay plates by UV cross linking. The first stage anti-adhesion specific mouse serum or hybridoma supernatant antibodies were diluted and incubated in the plate wells after the adhesions were bound and the plates blocked with BSA. After washing to remove non-specifically bound first stage reagents, a peroxidase coupled anti-mouse second stage antibody was added to the test wells, incubated, and washed. Substrate was then added to the wells to develop the assay which was subsequently read by an automated ELISA plate reader. If the desired sensitivity was not achieved, a third stage to amplify signals was employed.

Hybridoma fusion protocol - Anti-adhesion and anti-verotoxin hybridomas were subcloned and selected: 1) When hybridoma cell numbers permit, subcloning by limiting dilution in "HAT" medium is employed. 2) Upon successful ELISA screening of mAb-positive subclones, the hybridomas were adapted to serum-free medium for bulk production of anti-adhesion mAbs.

### EXAMPLE 6

### Pathogen-target Cell Interactions

The analyses of pathogen-target cell shear dependent interactions were conducted as described below. Leukocyte/endothelial cell interactions, were used as the model system for these studies.

Preparation of target cells - Freshly isolated human umbilical-cord endothelial cells (HUVEC) and commercially purchased HUVEC, which are Factor VIII and LDL-receptor pcsitive [cultured in endothelial-cell growth media (Clonetecs, EGM)), or E-selectin cDNA transfectants were grown to confluence on the internal surface of sterile glass 1.36 mm diameter capillary tubes (Drummond Scientific, Broomall, Penn) at least 24 hours prior to shear experiments. Four hours prior to the assay, the endothelial cells were treated with IL-1 (1 unit/ml) to induce E-selectin and other adhesion molecule expression.

Intestinal epithelial cells were isolated from bovine fetuses by isolation and dissection of the small intestine followed by treatment with trypsin-EDTA in Hanks balanced salt solution. Cells were plated and grown to confluence in T-25 flask, collected and frozen in liquid nitrogen. Cells were tested for their phenotype using anti-bovine monoclonal antibodies previously shown the be specific for bovine epithelium and shown to be greater than 95% epithelial cells.

Epithelial cells were thawed and plated into borosilicate capillary tubes (1.3mm internal diameter) in epithelial cell growth medium and allowed to grow to confluence. Epithelial cell containing capillary tubes were either activated with 100mM PMA for four hours or not activated before incorporation into the closed recirculating shear loop system.

Conduct of the shear assay ― Tubing was attached to the ends of the glass capillary tube to form a closed loop in which media and cells were recirculated; the tube was then mounted on an inverted microscope. Using a variable speed peristaltic pump, flow was regulated to simulate *in vivo* flow shear conditions (1-3 dynes/cm²) . The circulation loop permits multiple infusions of various mAbs during the continuous recirculation of leukocytes across the interactive surface of the HUVEC. The inverted microscope video-capture system, employing a mechanical stage, permited the survey of the entire length of the target cell monolayer and high resolution phase contrast recording of the interactive field for subsequent analysis. Adherence of bead-bound or planktonic forms of microorganisms to monolayers was established and continuously monitored for at least 10 minutes while being videotaped; during that time, control or experimental conditions were established and maintained. The cell interactions were observed and videotaped for an additional 10 min. The number of cells rolling on the activated BUVECs or epithelial cells was monitored before and after the injection of adhesion modifiers and determined by individual frame analysis of the recording. Data were recorded as the number of rolling cells within the view versus time. Parameters including rolling speed and rolling behavior were also analyzed.

### EXAMPLE 7

### Phage Affinity Purification of Anti-bacterial Peptide

Affinity purification of phage bearing epitopes bound by mAbs was performed as follows: 1 x 10¹² phage from a phage nonapeptide display library was combined with 1.0 ml of Sepharose beads conjugated with 4 mg of anti-adhesion blocking mAbs. The beads were mixed with the phage at 4°C for 16 hours by gentle inversion. The mixture was then loaded into a 5 ml plastic column barrel (Evergreen) and unbound phage removed by washing with 50 ml phage buffer (50 mM Tris-Cl pH 7.5, 150 mM NaCl, 0.5% Tween 20 (v/v), 1 mg/ml BSA). Bound phage were eluted from the column with 2.0 ml of eluting buffer (0.1 M glycine, pH 2.2), and the pH of the eluate was neutralized immediately with four drops of 2 M Trizma base. The titer of phage (nonapeptide library) was determined for each column eluate by plaque assay according to standard procedures. The column matrices were preserved for reuse in second and third round affinity purifications by washing with 10 ml PBS pH 7.0, followed by 3.0 ml PBS containing 0.02% sodium azide. The column was stored at 4°C until the next affinity purification, and was prepared for reuse by rinsing with 20 ml of phage buffer prior to mixing with amplified phage. As a control for antibody-specific selection, one column was prepared without antibody bound to the beads; all steps of affinity purification of phage were carried out on this control column and a sample of the resulting phage was sequenced to provide data defining the peptide sequences recognized by anti-SE or PA adhesion blocking mAbs.

### EXAMPLE 8

### ADHERENCE OF E. COLI ON BOVINE INTESTINAL CELLS

Currently, the detection and identification of VTEC in meat samples is based on conventional cultural and serological methods which are labor intensive and technically difficult. The pre-harvest detection of VTEC organisms in food animals appears to be the most effective means of diminishing the incidence of food-borne enteric disease approach to developing a highly specific diagnostic tool exploits the antigenic properties of two virulence factors that are shared by all VTEC, namely, the Shiga-like toxin (slt), sometimes referred to as verotoxin, and adhesion proteins (adhesions) that promote specific interactions with enterocytes. The inventors isolate and identify VTEC in fecal specimens using a diagnostic assay that simultaneously "captures" and identifies dead or viable VTEC microorganisms in clinical samples or feces specimens. The detection reagents used in the assay were monoclonal antibodies against adhesion epitope(s) and shiga-like toxins (slt) of VTEC.

The rationale for the approach is based on numerous observations (4, 5, 6, 7, 8) that two events promoted by VTEC virulence factors are characteristic of VTEC-induced enteric disease. These are adhesion-mediated attachment to and effacing (A/E) of microvilli of the large intestine and verotoxin-induced hemorrhage and damage to the kidney. As virulence factors, the VTEC adhesions and verotoxins are antigenic and represent highly specific markers for an antibody-based assay technology. VTEC possess two antigenically distinct adhesion proteins which operate together or singly to bring about attachment of VTEC to microvilli. The first adherence protein is a fimbrial gene product of plasmid encoded loci referred to as the bundle forming pilus (3FP) . The fimbrial adhesion appears to promote non-intimate or localized adherence to the microvilli. The second, a 94-kDa outer membrane protein (OMP) referred to as the OMP adhesion promotes intimate contact with enterocytes.

Materials and Methods - Plain immunomagnetic beads were obtained commercially, e.g. from Dynal, and coated with adhesion or SLT mAbs using a protocol recommended by Dynal. In essence, the beads were mixed with an appropriate amount of mAb for 30 min. at 4°C, collected in a magnetic particle concentrator, washed 5 times and finally suspended in buffer. Various concentrations of VTEC suspended in phosphate-buffered saline (PBS) were reacted with the coated beads to determine the capture efficiency of the beads. Cells attached to the beads were stained with DAPI and examined using epifl uorescence microscopy to determine the number of attached cells. To detect slt in broth culture supernatant fluids, magnetic beads coated with anti-slt were used to capture free sit and identified using a sandwiching technique involving a follow-on fluorescent labeled anti-slt.

Suspensions of *E. coli* strains, both 3A (from R.A. Wilson, Penn State University) and 932 (from U.S. E.P.A.), were grown on and harvested from blood agar plates and tryptone medium and used live or fixed in formalin for adherence to magnetized beads and for the conduct of the shear analyses. Upon incorporation of the capillary tube lined with bovine epithelial cells, (see above) in the loop system, a flow-induced shear force was established as a pulsatory wave flow peaking at 2 dynes, using Hanks balanced salt solution, HEPES (pH 7.0) as the medium. *E.* coli coated beads, suspended in PBS were infused into the shear flow via an injection port. Inceractions of *E. coli* coated beads with epithelial cells were observed by video microscopy and recorded to video tape as a permanent record and for off-line computer image analysis. Adhesion of bead-bound E. coli was analyzed for types of adhesive interactions (rolling or sticking), characteristics (single beads or aggregates), and numbers of adhesive interactions over an interaction interval of ten minutes for the analysis of each condition. Hybridomas and monoclonal antibodies have been produced from spleen cells of immunized mice that recognize various cell surface determinants of the 0157 strain of *E. coli.* The demonstration of shear dependent adhesion of 0157:H7 tc bovine intestinal cells confirmed the presence and function of microbial attachment molecules and ligand structures on bovine cells. Adherence was dependent on culture conditions (growth on blood agar) for the organism that appeared to promote the development of adhesions. Sera recognize antigenic differences promoted by cultural conditions in "broth grown or blood agar grown" 0157 *E.* coli. This data suggests that blood agar influences the development of specific cell surface adhesions recognized by these antibodies not found in cells grown in broth. Capture of *E. coli* 0157:H7 from suspension is dependant on the type and quantity of magnetic particle used, as well as the incubation procedures. Up to 98% of the cells in a 1x10⁵ CFU/ml can be captured under laboratory conditions using these methods. Only 60% of attached cells were removed from the magnetic particles by elution agents. Nearly 100 percent of 0157 inoculated into ground beef samples were detected by a refinement of the immunomagnetic separation (IMS) procedure.

The inventors have identifed several peptides and their cDNA sequences which are incorporated into a vaccine delivery system. These peptides include a) peptide 11 with an amino acid sequence of KPHTHKHKV which mimics an adhesive region of the B oligomer of the verotoxin of E. coli C157:H7; and b) peptide DG with an amino acid sequence of DDTFTVKVDG which mimics another adhesive region of the B cligomer of E. coli 0157:H7.

### References for Example 8

1. Karmali, M.A.., 1989. Infection by verocytotoxin-producing *Escherichia coli*. Clin. Microbiol. Rev. 2:15-38.
2. Wells, J. G., L. D. Shipman, K. D. Greene, E. G. Sowers, J. H. Green, D. N. cameron, F. P. Downes, M. L. Martin, P. M. Griffin, S. M. Ostroff, M. E. Patter, R. V. Tauxe, and I. K. Wachsmuth. 1991. Isolation of *Escherichia coli* serotype 0157:H7 and other shiga-lik toxin-producing *E. coli* from dairy cartle. J. Clin. Microbiol. 29: 985-989.
3. Doyle, M. P., and J. L. Schoeni. 1987. Isolation of *Escherichia coli* 0157:H7 from retail fresh meat and poultry. Appl. Environ. Microbiol. 53: 2394-2396.
4. Pyle, B. H., S. C. Broadway, and G. A. McFeters. 1995. A rapid, direct method for enumerating respiring *Escherichia coli* in water. Appl. Environ. Microbiol. 61: 2614-2619.
5. Junkins, A. D. and M. P. Doyle. 1989. Comparison of adherence properties of *Escherichia coli* 0157:H7 and a 60-mega-dalton plasmid-cured derivative. Cirr. Microbiol. 19:21-27.
6. Tzipori, S., R. Gibson, J. Montanareo. 1989. Nature and distribution of mucosal lesions associated with enteropathogenic and enterohemorrhagic *Escherichia coli* serotypes in piglets and the role of plasmid-mediated factors. Infect . Immun. 57:1142-1150.
7. Tesh, V.L. and A.D. O'Brien. 1992. Adherence and colonization mechanisms of enteropathogenic and enterohemorrhagic *Escherichia coli.* Microb. Pathog. 12: 245-254.
8. Louie, M., J. DeAzavedo, R. Clarke, A. Borczyk, H. Lior, M. Richter and J. Brunton. 1994. Sequence heterogeneity of the eae gene and detection of verotoxin-producing *Escherichia coli* using serotype-specific primers. Epidemiol. Infect. 112: 449-461.

### EXAMPLE 9

### ADHERENCE MOLECULES OF CANDIDA ALBICANS AND THEIR DETECTION IN CLINICAL SPECIMENS

Candida albicans is the most common cause of opportunistic fungal diseases in humans and has become the fourth leading cause of cases of nosocomial blood stream infections (15). An important step in the pathogenesis of disseminated candidiasis appears to relate to the ability of Candida cells to adhere to specific tissue locations within the host (7). The search for the appropriate fungal molecules is not trivial, however, as it has been shown that the surface antigenic makeup of C. albicans, is variable both in vitro (3,4,5) and *in vivo* (6). Recently, Cutler *et al.* isolated the candidal adhesions responsible for adherence of C. *albicans* yeast cells to murine splenic marginal zone macrophages and macrophages in specific areas of peripheral lymph nodes (10). The adhesins are part of the mannan portion (12) of the phosphomannoprotein complex (PMC) and represent a major part of the cell surface. One adhesin has been identified as a β-1,2-linked tetramannose located in the acid-labile part of the PMC and another adhesin resides in the acid-stable part of the complex (11). Detection of mannan adhesins in the serum of patients will thus prove to be a reliable indicator of disease. The adhesins are produced in abundance during growth of the fungus and are readily shed from the cell surface. Using high affinity monoclonal antibodies specific for candidal adhesion moieties, leads to the development of a rapid and reliable test for disseminated candidiasis under the invention.

The presence of fungal adhesins in the serum of patients would indicate active disease and not colonization. Since candidal mannan is spontaneously released during growth of the fungus, assays are designed to detect this polysaccharide (8).

The approach to a diagnostic solution resides with the early and rapid detection of candida: antigens in the body fluids or cells of patients with highly specific monoclonal antibodies (mAbs) directed against candidal antigens shed or released by the organism as it begins to grow and traffic in the body. The invention tests a panel of mAbs specific for an array of candidal cell surface antigens for the detection of candidal antigens in infected mice and humans. The development and use of anti-adhesion monoclonal antibodies and monoclonal antibodies directed to undefined cell surface determinants shed by or extracted from *Candida albicans* in vitro may be for the detection of candidal antigens in the body fluids and cells of infected mice and humans. The inventors developed a Candida peptide with an amino acid sequence which mimics a region of the phosphomannoprotein adhesion of *C. albicans.*

### Development of an ELISA test to detect Candida antigens

The invention provides a key assay to detect the presence of Candida adhesions in serum and on host cells . The assay uses an antibody capture/sandwich ELISA method. Capture antibodies specific for Candida albicans antigens were used to coat the surface of polystyrene microtiter plates. After appropriate washing away of unbound capture antibody and blocking of unreacted sites on the plastic, test serum that contain Candida antigens was added to the antibody-coated wells, incubated and washed to remove unbound serum. A secondary antibody specific for the antigen was added to the wells, allowed to react with antigen bound to the capture antibodies and washed to remove all unbound materials. A tertiary antibody, which is specific for the secondary antibody and to which an appropriate enzyme is covalently coupled, was added to the wells to allow binding to all secondary antibody in the wells. After washing away unbound tertiary antibody, an appropriate chromogenic substrate specific for the enzyme on the tertiary antibody was added to the wells. As compared to appropriate positive and negative controls, a color reaction in the wells allows determination of the presence of Candida antigen in the original test serum.

Once the ELISA assay was optimized to detect the minimum amount of *Candida* antigen in antigen-spiked normal mouse serum, serum from humans and mice with various degrees of hematogenous disseminated candidiasis were examined.

### Detection of Candidal Soluble Adhesins on Mouse and Human "Buffy Coat" Cells and Serum.

The attachment of shed candidal cell surface determinants to peripheral white blood cells early in infections with C. albicans serves as the basis for a rapid diagnostic test using the buffy coat cells of freshly drawn blood from the infected patient.

Anti-adhesion mAbs and mAbs were developed to detect and determine the cellular distribution of soluble candidal cell surface determinants adherent to (a) peripheral blood leukocytes represented in the "buffy coat" layer of blood from infected mice *(in vivo* binding), (b) antigen-spiked human and mouse buffy coat cells *(in vitro* binding) and c) samples of human serum from patients infected with C. *albicans* using existing and a new mAbs. The distribution of candidal antigen on buffy coat leukocytes was determined by coupling the analysis of cell population with flow cytometry and detection of antigen with immunofluorescence or ELISA techniques.

The panel of antibody reagents may be used in detecting shed cell surface antigens in mice infected with C. albicans. Vaccine preparations containing candidal cell surface determinants were prepared from chemical extracts of C. *albicans* or supernatants of Candida cultures and used to immunize mice. Candidal cell surface vaccines were prepared from a) Shed cell surface determinants in supernatants of C. albicans cultures, b) EDTA extracts of C. albicans, and c) 2-ME (2-mercaptoethanol) extracts of C. *albicans.*

Hybridomas were prepared from spleen cells of animals making high titer polyclonal antibody responses to *C. albicans* cell surface determinants. The technique for preparing hybridomas and producing monoclonal antibodies are described above.

The results of studies with monoclonal antibodies directed against candidal adhesins have demonstrated that phosphomannoprotein (PMP) adhesins shed by *Candida* can be detected in serum in the 1-10 ng range. Moreover, another study on candidal antigenemia has shown that candidal adhesins can be detected in 70.4 percent of infected mice even in cases where blood cultures for C. *albicans* were negative. These studies provide the basis for a highly sensitive diagnostic assay for candidal infections.

The inventors have successfully produced a peptide mimic of a key adhesive domain of the PMP adhesin by probing a phage display library with a monoclonal antibody preparation that has been shown to passively immunize mice to challenge with *C. candida.* The peptide appears to block the attachment of C. albicans to host target cells. Currently, oligonucleotide sequences are being prepared from the peptide sequence data and are being incorporated into a plasmid construct for insertion into a live vector (*e.g.*, an attentuated *Salmonella typhimurium or Shigella* strain). The resultant recombinant vaccine is given orally to adult mice followed by assays of mucosal and systemic immune responses.

### REFERENCES EXAMPLE 9

1. **Anttila, V. J., P . Ruutu, S. Bondestam, S. E. Jansson, S. Nordling, M. F'arkkil'a, A. Sivonen, M. Castren, and T. Ruutu.** 1994. Hepacosplenic yeast infection in patients with acute leukemia: a diagnostic problem. Clin. Infect. Dis. 18:979-981.
2. Berenguer, J., M. Buck, F. Witebsky, F. Stock, P. A. Pizzo, and T. J. Walsh. 1993. Lysis-centrifugation blood cultures in the detection of tissue-proven invasive candidiasis. Diagn. Microbiol. Infect. Dis. 17:103-109.
3. Brawner, D. L. and J. E. Cutler. 1984. Variability in expression of a cell surface determinant on Candida albicans as evidenced by an agglutinating monoclonal antibody. Infect. Immun. 43:966-972.
4. **Brawner, D. L. and J. E. Cutler.** 1986. Variability in expression of cell surface antigens of *Candida albicans* during morphogenesis. Infect. Immun. 51:337-343.
5. **Brawner, D. L. and J. E. Cutler.** 1986. Ultrastructural and biochemical studies of two dynamically expressed cell surface determinants on *Candida albicans.* Infect. Immun. 51:327-336.
6. **Brawner, D. L. and J. E. Cutler.** 1987. Cell surface and intracellular expression of two Candida *albicans* antigens during in vitro and in vivo growth. Microbial Pathogen. 2:249-257.
7. **Cutler, J.E.**1991. Putative virulence factors of *C. albicans.* Ann. Rev. Microbiol.**45**:187-218.
8. **de Repentigny, L., R. J. Kuykendall, F. W. Chandler, J.R. Broderson, and E. Reiss.** 1984. Comparison of serum mannan, arabinitol, and mannose in experimental disseminated candidiasis. J. Clir.. Microbiol. 19:804-812.
9. **Han, Y. and J.E. Cutler.**1995. Antibody response that protects against disseminated candidiasis. Infect. Immun.63:2714-2719.
10. Han, Y., N. van Rooijen, and J. E. Cutler. 1993. Binding of *Candida albicans* yeast cells to mouse popliteal lymph node tissue is mediated by macrophages. Infect. Immun. 61:3244-3249.
11.Kanbe, T. and J. E. Cutler. 1994. Evidence for adhesion activity in the acid-stable moiety of the phosphomannoprotein complex of Candida *albicans.* Infect. Immun. 62:1662-1668.

### EXAMPLE 10

### THE CHARACTERIZATION OF ADHERENCE OF HANTAVIRUS TO VERO E6 CELLS

At present, there are no satisfactory diagnostic tests to detect or vaccines to prevent Hantavirus infections in man. Hantavirus attaches to cells during the infectious process by recognition of a cell receptor by a viral anti-receptor. The identities of the cellular receptor for hantavirus and the anti-receptor are unknown. The identification of both entities are particularly important for the development of anti-viral reagents and potental vaccines. The inventors identify and characterize these structures using a novel in vitro analytical approach to the characterization of adherence events and the pathogenesis of hantavirus infections. Molecular Cloning: The small (S) and medium (M) genomes of the virus(es) detected are cloned using standard procedures (5) and the primers (M-3'(+), M-5'(-), S-3'(+), S-5'(-), M-OP (+), etc.) are used.

The M and S primers discovered by the inventors are used for the cloning cf the Hantavirus small and medium genomes, respectively. The cDNAs are subclcned into E. *coli* or *Baculovirus* vectors (microbial transfectants) for expression. The pRSET and pTRCHis kits supplied by Invitrogen for expression in E. coli and Invitrogen's pBluBacHis kit for expression in Baculovirus are used. The recombinant proteins are purified by metal chelate chromatography (Invitrogen) . The envelope glycoproteins G1 and G2 of Hantavirus serves as the targets for studies on adhesion to host cells.

Preparation of monoclonal Antibody: Mice are used for the preparation of monoclonal antibody using recombinant antigens prepared by expression in *E. coli* or Baculovirus. No live virus is used to infect or immunize animals in containment. BALB/c mice are immunized subcutaneously with recombinant protein (G1, G2 or N) suspended in PBS and emulsified in CytRx TiterMax #R-1 at a 50/50 ratio.

Antibody Detection: All of the antibody detection has been done by the ELISA developed by the Centers for Disease Control (CDC) for detection of Sin Nombre virus. This procedure is semi-quantitative and detects only antibody to nucleocapsid antigen (N). The assay may be expanded by employing recombinant antigens representative of the glycoproteins G1 and G2 in the standard ELISA. The envelope proteins are thought to be important in neutralization of viral infectivity.

Adhesion assay: The analyses of adhesion events that seek to define the nature and function of Hantavirus receptor and anti-receptor molecules involve the use of recombinant antigens expressed in Escherichia coli with Vero E6 target cells. *E.* coli expressing the envelope glycoproteins G1 and G2 or the nucleocapsid antigen (N) is reacted with paramagnetic beads coated with mAb directed against G1, G2 or N proteins and examined under shear in accord with the procedures described herein.

### REFERENCES EXAMPLE 10

1. Duchin, J. S., F. T. Koster, C. J. Peters, G. L. Simpson, B. Tempest, S. R. Zaki, T. G. Ksiazek, P. E. Rollin, S. Nichol, E. T. Umland, R. Moolenaar, S. Reef, K. Nolte, M. Gallaher, J. Butler, and R. Breiman. 1994. Hantavirus pulmonary syndrome: a clinical description of 17 patients with a newly recognized disease.. N. Engl. J. Med. 330:949-955.
2. **Foucar, K., K. B. Nolte, R. M. Feddersen, B. Hjelle, S. Jenison, J. McLaughlin, D. A. Madar, S. A. Young, S. R. Zaki, and L. Hughes.** 1994. Outbreak of Hantavirus pulmonary syndrome in the southwestern United States. Response of pathologists and other laboratorians. Am. J. Clin. Pathol. 101:S1-58.
3. **Nichol, S. T., C. Spiropoulou, S. Morzunov, P. Rollin, T. Ksiazek, H. Feldmann, A. Sanchez, J. Childs, S. Zaki, and C. J. Peters.** 1993. Genetic identification of a hantavirus associated with an outbreak of acute respiratory illness. Science 262:914-917.
4. **Childs, J. E., T. G. Ksiazek, C. F. Spiropoulou, J. W. Krebs, S. Morzunov, G. O. Maupin, K. L. Gage, P. E. Rollin, J. Sarisky, R. E. Enscore, J. Frey, C. J. Peters, and S. Nichol.** 1994. Serologic and genetic identification of Peromyscus maniculatus as the primary rodent reservoir for a new hantavirus in the SW United States. J. Infect. Dis. 169:1271-1280.
5. **Ausubel, F., R. Brent, R. Kingston, D. Moore, J. Seidman, J. Smith, and K. Struhl.** 1995. Current Protocols in Molecular Biology. Greene Publishing Associates and Wiley-Interscience, New York.

### EXAMPLE 11

### ADHERENCE OF TRITRICHOMONAS FOETUS TO BOVINE TARGET CELLS

*Trichomonad biology* and disease - *T. foetus* is an important protozoan pathogen of beef cattle throughout the U.S. (1) causing considerable prenatal death and transient infertility, and therefore substantial economic losses. This parasite is restricted to the reproductive tract of cattle and has but one morphologic form, the trophozoite, which remains on the mucosal surfaces of the reproductive tract, except in the pregnant animal, where it invades the placenta. T. foetus can also invade fetal tissues such as the lung, intestinal epithelium and subscapular lymph nodes (4). These results as well as those from *in vitro* experiments (2,3) suggest that *T. foetus* attaches to tissue and cell surfaces which enables the parasite to subsequently invade such tissues.

The inventors have demonstrated that *T. foetus* can kill mammalian targets and lyse red cells (2) and surface-reactive antibodies against *T. foetus* protect against cytotoxicity and block adhesion of *T. foetus* to the target cells (3). Further results show enhanced cytotoxicity by highly adherent clones as compared to poorly adherent clones of *T. foecus* (3) These results indicate that the ability of *T. foetus* to adhere to targets give the parasite efficient cytotoxic capability. These results also suggest that one mechanism by which antibody could protect bovine tissue from damage is by interfering with adherence and/or some other contact-dependent aspect of cytotoxicity. Data from reactions of a surface-reactive, inhibitory mAb (32.3B3.4) with Western blots of whole *T. foetus* indicate that this mAb recognizes a band of approximately 190,000 MW on nonreduced gels and bands at 140,000 and 60,000 on reduced gels (3) , a molecule the inventors have termed Tf190. Thus high molecular weight surface molecules that are immunogenic in cattle (5) and mice (3), are involved in adhesion and/or cytotoxicity toward mammalian cells.

The inventors evaluate the effects of antibodies specific for Tf190 on adhesion of T. foecus to mammalian cells. Cattle are immunized with affinity-purified Tf190 and the anti-adhesion properties of antibodies from these cattle are evaluated using the MIT Shear assay described herein. Additional mAbs reactive with purified Tf190 are identified and their effeccs on adhesion of *T. foetus* to mammalian cells are evaluated. Additional mAbs are prepared against purified Tf190 and screened for their effects on parasite adhesion as well. The specificity of antibodies and mAbs for Tf190 is established by dot immunobinding assay on purified Tf190 and reaction of antibodies with Western blots of whole extracts of T. foetus and Tf190 (3).

### REFERENCES EXAMPLE 11

1. BonDurant, R. H., and B. M. Honigberg. 1994. Trichomonads of Veterinary Importance. In, "Parasitic Protozoa", 2nd edition, J. P. Kreier, ed., 9:111-188.
2. Burgess, D. E., K. F. Knoblock, T. Daugherty and N. P. Robertson. 1990. Cytotoxic and hemolytic effects of *Tritrichomonas foetus* on mammalian cells. Infec. Immun. 58:3627-3632.
3. Burgess, D. E., and C. M. McDonald. 1992. Analysis of adhesion and cytotoxicity of *Tritrichomonas foetus* towards mammalian cells with monoclonal antibodies. Infection and Immunity 60:4253-4259.
4. Rhyan, J. C., K. L. Wilson and D. E. Burgess. 1994. Immunochemical detection of T. foetus in formalin-fixed paraffin-embedded sections of bovine placenta and fetal lung. J. Vet. Diagn. Invest. 25: 350-355.
5. Corbeil, L. B. *,* J. L. Hodson, D. w. Jones, R. R. Corbeil, F. R. Widders and L. R. Stephens. 1989. Adherence of Tricrichomonas foetus to bovine vaginal epithelial cells. Inf. Immun. 57:2158-2165.
6. Huang, J-C, D. Hanks, W. Kvasnicka, M. Hanks and M. R. Hall. 1989. Antigenic relationships among field **isolates of Tritrichomonas foetus from cattle . Amer. J. Vet. Res.** 50:1064-1068.

### EXAMPLE 12

### MATERIALS AND METHODS FOR THE PREPARATION OF SUBUNIT COMPONENTS OF MICROBIAL ADHESION MOLECULES AND THEIR COUNTER-RECEPTORS (LICANDS) ON HOST CELLS FOR USE AS DIAGNOSTIC REAGENTS, THERAPEUTIC COMPOUNDS AND VACCINE MATERIALS.

The following procedures have been developed or adopted by the inventors to prepare subunit components of adhesive domains or sites including flanking domains of microbial attachment molecules for use as a therapeutic (blocking) compound dr for insertion into recombinant vaccines . The use of multiple peptide domains within and around the attachment site in a vaccine formulation ensures the incorporation of antigenic peptides that elicit an immune response in the host. The materials and methods described below are applied to the development of adhesive diagnostic reagents, therapeutic peptides and carbohydrates and recombinant subunit vaccines for any of the four classes of pacthogenic microorganisms represented in the claims.

### Detection and characterization of microbial adhesion systems.

The isolation of a pathogen's adhesin (attachment) molecule **(PAM)** which mimics the adhesion molecule expressed on host cells begins with its detection and characterization in the inventors *in vitro* shear assay system using target cells that express the ligand for the adhesion molecule or purified ligands coated on the lumenal surface of a capillary tube reaction chamber. The technique for establishing various adhesive substrates on the internal surface of small capillary tubes for the analyses under high and low physiological shear forces of any type of cell-cell adhesive interaction has been developed and perfected by the inventors (Bargatze, R., *et al* . 1994. J. Immunol. 152: 5814-5825; Jutila, M., *et al*. 1994. J. Immunol. 153: 3917-3928; Bargatze, R., *et al* 1994. J. Exp. Med. 180: 785-792).

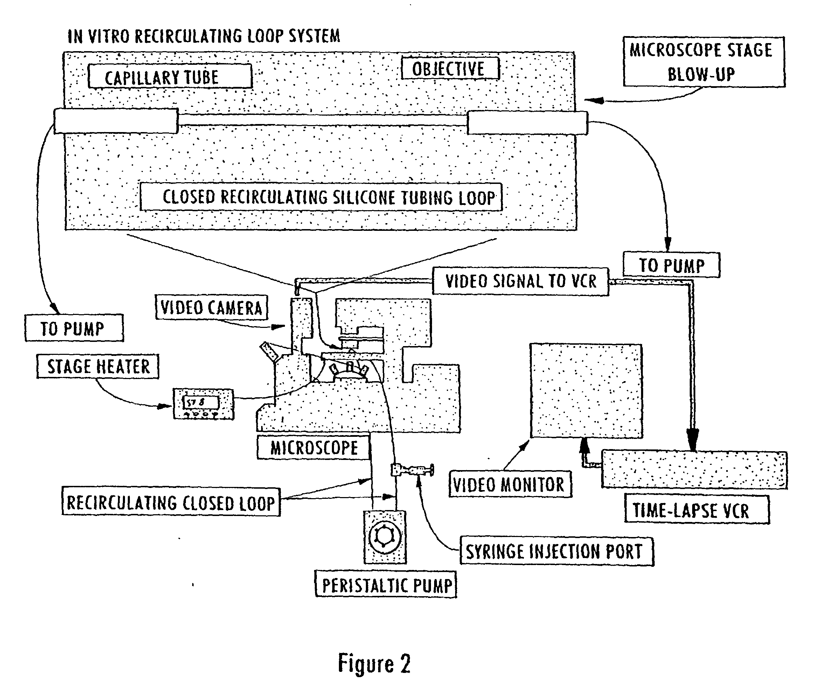

The detection and characterization of PAMs and their ligands involves the use of an analytical in vitro shear flow apparatus (See Figure 2) that provides a reproducible real-time monitoring, and quantification of microbial adhesive interactions with a panel of ligand assemblies displayed on immobilized substrates or cells of the endothelia and epithelia or other target cells during simulated physiological shear. The system consists of a closed capillary loop in which fluids are recirculated via a peristaltic pump. The infusion of cells injected into the loop brings about an encounter with monolayers of target cells coating the internal surface of the capillary tubes reminiscent of blood flow in the vascular system. The subsequent adhesive interactions of microbial cells and target cells or ligand bearing substrates are analyzed with the use of a professional-grade video data acquisition and recording system. The Macintosh® computer system (Apple Computer®, INC., Cupertino, CA), LG-3 video frame grabber board, (Scion Corporation, Frederick, MD), and Image software (NIH, Bethesda, MD) are customized for real-time and off-line video analysis of adhesion events.

The analyses of pathogen-target cell shear dependent interactions are conducted as described below. Leukocyte/endothelial cell interactions, with which the inventors have extensive experience, are used as the model system for these studies.

An in vivo shear model employing intravital microscopy has also been developed by the inventors to examine cellular interactions under physiological shear forces typical of those found in the venules of the intact animal. The interaction of transfused FITC-labeled organisms with high endothelial venules in exteriorized mouse Peyer patches or intestinal epithelium can be observed and recorded. For microbial adhesion to target cells, a static adhesion assay can also be conducted on tissue sections or tissue culture preparations of established cell lines or cells from any organ system.

### EXAMPLE 13

### Preparation of immobilized target cells or Pathogen screening matrices

Immobilized Target cells.- Freshly isolated human umbilical-cord endothelial cells (HUVEC) which are Factor VIII and LDL-receptor positive [cultured in endothelial-cell growth media (Clonetecs, GGM)], or specialized cell lines of HUVEC expressing tissue specific addressins, or receptor cDNA transfectants are grown to confluence on the internal surface of sterile glass 1.36 mm diameter capillary tubes (Drummond Scientific, Broomall, Penn) at least 24 hours prior to shear experiments. Four hours prior to the assay, the endothelial cells are treated with IL-1 (1 unit/ml) to induce E-selectin, or interferon and interleukin-1 to induce ICAMs and VCAM.

Intestinal epithelial cells are freshly isolated from human umbilical cord or bovine fetuses by dissection of the small intestine followed by treatment with trypsin-EDTA in Hanks balanced salt solution. Alternatively, the human epithelial cell lines Caco-2 (colon) or Int-407, (jejunum/ileum) are used. The Caco-2 cell line has been shown to bind *Salmonella typhi* (Tartera, C., 1995. Infect. Immun. 61: 3084-3089.), *Escherichia coli* (Fratamico, P. *et al*. 1993. J. Med. Micro. 39: 371-381), Aeromonas (Nishikawa,, Y., et al. 1994. J. Med. Micro. 40: 55-61. and *Kelbsiella pneumoniae* (Favre-Bonte, S., et al. 63: 1318-1328.). Cacc-2 and Int-407 cells are grown in RPMI 1640 medium (Sarem, F. et *al* 1996. Appl. Micro 22: 439-442) and are used at late confluence after 5 days for Int-407 and at the differentiated stage for Caco-2 after 21 days of incubation at 37°C in 5%CO2/95% air atmosphere. Freshly isolated epithelial cells are plated and grown to confluence in T-25 flask, collected and frozen in liquid nitrogen. Cells are tested for their phenotype using anti-epithelial cell monoclonal antibodies previously shown the be specific for human or bovine epithelium and shown to be greater than 95% epithelial cells. Epithelial cells are thawed and plated into borasilicate capillary tubes (1.3mm internal diameter) in epithelial cell growth medium and allowed to grow to confluence. Capillary tubes containing confluent monolayers of epithelial cells are either activated with 100mM PMA or integrin activators. (chemokines) for four hours or not activated before incorporation into the closed recirculating shear loop system.

### EXAMPLE 14

PAM screening matrices. - Matrices displaying physiological ligands for PAMs are prepared from known carbohydrate and glycoprotein counter-receptors that bind cellular receptors used by pathogenic microbes. Various macromolecular structures have been designed by the inventors for use as diagnostic materials for the rapid screening of pathogens for PAMs. For developing carbohydrate-terminated matrices, the inventors use a method (Method 1) described by Spevak et al (Spevak, W., *et al.* 1996. J. Med. Chem. 39:1018-1020) for the construction of acidic multivalent assemblies displaying carbohydrate ligands for detecting or identifying PAMs in the shear assay system. These matrices consist of an acidic lipid scaffolding supporting glycclipid ligands that are used to coat the internal walls of capillary tubes. Carbohydrate-terminated lipid matrices are made stable by polymerization with UV. Another Method (Method 2) consists of diluting the N-glycosides or glycolipid in methanol, drawing approximatley 100µl containing 100 ng glycolipid into a capillary tube and allowing the solvent to evaporate overnight at 37°C. The lumen of the tubes is then coated with 200µl of 2% bovine serum albumin (BSA) in phosphate buffered saline (PBS) for 2 hours, washed twice with the BSA solution, and finally flushed free of the coating solution. For developing glycoprotein-terminated matrices, a Method 3 includes incubation of glycoprotein adhesion molecule constructs in HBSS media (100µg/ml) containing Ca++ and Mg++ plus detergent for one hour at 37°C in capillary tubes. The tube is then cooled to 4°C for 15 minutes and flushed (washed) with HBSS medium maintained at 4°C. The inventors have developed an extensive panel of purified glycoproteins including E-L- and P-selectins, ICAM, VCAM, MAdCAM-1 and β1 and *β2* integrins. Using Methods 1, 2 or 3, the inventors have formulated several novel PAM screening matrices for use as diagnostic reagents in the shear assay system. These include but are not limited to the following.

| **PAM Screening matrix** cCompositton (coating cone.) | **PAM Detected (Example)** |
|---|---|
| **Carbohyrate-terminated** | |
| Galα-4GalβCer(100 ng) | *Shigella dysenteriae* (B peptide C-type lectin) |
| GalcNAc Cer (100 ng) | *Entamoeba histolytica* (Membrane C-type lectin) |
| GalNAc (1-4)GalCer (100 ng) | *Neisseria gonorrhoeae* (Pilin C-type lectin) |
| Fucosylated 0 blood group (100 ng) | *Helicobacter pylori* (Cell wall C-type lectin) |
| GlCNAcβ(1-3)Gal Cer (100 ng) | *Streptococcus pneumoniae* (cell wall C-type lectin) |
| Ga)Nacβ(1-4)Gal. NeuAC., GlcCer ( 100 ng) | *Escherichia coli* (Fimbrial CFA/I lectin) |
| Glycoprotein-terminated | |
| MAdCam-1 (100 µg) | α4β7-like receptor |
| ICAM (100 µg) | LFA-1-like receptor (Candida *albicans)* |
| CR3 integrin (100 µg) | RGD domain (Leishmania.) |
| CR3 integrin (100 µg) | RGD domain *(Bordetella pertussis)* |
| VCAM-1 (100 µg) | α4β1-like receptor |
| (IG domains of CD4 (100 µg) | AIDS virus receptor |
| IG domains of ICAM (100 µg) | LFA-1-like receptor (Rhinovirus) |
| | |
| Cer=Ceramide *YTBD=Yet to be discovered | |

### EXAMPLE 15

### The shear assay-

Plain immunomagnetic beads such as those from Dynal are coated with mAbs directed against cell surface adhesins or soluble adhesins (S-adhesin). The beads are mixed with an appropriate amount of mAb for 30 min. at 4°C, collected in a magnetic particle concentrator, washed 5 times and finally suspended in buffer. Suspensions of microorganisms are grown on and harvested from media shown to promote the development of adhesion molecules and fixed in formalin for adherence to magnetized beads and for the conduct of the shear analyses. Cells attached to the beads are stained with DAPI and examined using epifluorescence microscopy to determine the number of attached cells.

To detect and characterize the adherence of soluble adhesins (S-adhesins), largely exotoxins, from broth culture supernatant fluids, magnetic beads coated with anti-S-adhesins are also used to bind the S-adhesins and identified using a sandwiching technique involving a follow-on fluorescent labeled anti-S-adhesin. Upon incorporation of the capillary tube lined with human or animal target cells (normally endothelial cells and/or epithelial cells) into the loop system, a flow-induced shear force is established as a pulsatory wave flow peaking at 2 dynes using Hanks balanced salt solution, HEPES (pH 7.0) as the medium. Microbe-coated beads, suspended in PBS are infused into the shear flow via an injection port. Interactions of microbe-coated beads with epithelial cells are observed by video microscopy and recorded to video tape as a permanent record and for off-line computer image analysis. Adhesion of bead-bound microbes is analyzed for types of adhesive interactions (rolling or sticking), characteristics (single beads or aggregates), and numbers of adhesive interactions over an interaction interval of ten minutes for the analysis of each condition.

All studies on the blocking of adhesion interactions with anti-adhesin mAbs and peptides are conducted on the Montana ImmunoTech (MIT) ligocyte® pharmaceutical, inc. Shear Analysis System described herein.

The circulation loop permits multiple infusions of various specific adhesion- or ligand-blocking mAbs during the continuous recirculation of leukocytes across the interactive surface of the HUVEC. The inverted microscope video-capture system, employing a mechanical stage, permitted the survey of the entire length of the targe= cell monolayer and high resolution phase contrast recording of the interactive field for subsequent analysis. Adherence of bead-bound or planktonic forms of microorganisms tc monolayers is established and continuously monitored for at least 10 minutes while being videotaped; during that time, control or experimental conditions were established and maintained. The cell interactions are observed and videotaped for an additional 10 min. The number of cells rolling on the activated endothelial or epithelial cells is monitored before and after the injection of adhesion modifiers and determined by individual frame analysis of the recording. Data are recorded as the number of rolling cells within the view versus time . Parameters including rolling speed and rolling behavior are also analyzed. These observations taken together serve to define microbial adhesion specificities for ligand structures on host tissues and cells and permits the inventors to test various reagents and modify adhesive interactions under physiological conditions.

### EXAMPLE 16

### PAM Screening Matrices as Diagnostics for Detecting and Characterizing Elements of or Participants in Microbial/Target Cell (Ligand) Interactions.

### Development of Diagnostic Probes for Identifying the Structure of PAMs : Monoclonal antibody reagents.

The preparation of a vaccines, diagnostic reagents or therapeutic peptides containing the elements of the adhesive domain (site) of PAMs and/or their ligands continues with the development of monoclonal antibodies (mAbs) directed against different regions of the attachment molecule detected in the Shear System. Monoclonal antibodies are developed in accord with well established techniques for defecting and characterizing adhesion systems involved in leukocyte/endothelial interactions reported in prior publications (Berg, E. L. *et al.* 1993. Nature 366:695-698. and Jutila, M. 1994. Adv. Pharmacol. 25: 235-262). Hybridomas producing anti-adhesion antibodies are typically prepared from mouse spleen-derived B-cells immunized with either adhesin positive, intact microbial cells or purified adhesin material prepared from cell wall extracts (Brauwner, D. L. and J. E. Cutler, 1986. Infect. Immun. 51: 327-336.) of adhesin-positive microbes. Monoclonal antibodies are produced against ligand structures on host cells, both glycoprotein and glycolipid in nature, to develop reagents for detecting and blocking microbial adhesion events and to serve as probes for use in the phage display library described below. Many glycoconjugates used by the inventors are commercially available. The specificity and blocking properties of mAbs for the microbe/host target cell interactions are analyzed using the *in vitro* shear assay system described above.

### EXAMPLE 17

Immunization protocol: Balb/c mice, ranging in age from 8 to 12 weeks, are used for hybridoma and antibody production. Primary immunization antigens are adjusted to 10-20 µg of soluble adhesion molecule or glycoconjugates suspended in .5ml PBS or 5 x 10⁶ or 5 x 10⁷ formalin-treated cells are emulsified in CytRx*® TiterMax*® #R-1 at a 50/50 ratio. In all cases, the subcutaneous (SC) route is used to avoid a toxic reaction in mice . Immunized mice are bled at day 14 to test antibody responses by ELISA or agglutination of intact cells. If a low titer is found, the mice are boosted with similar dose of antigen and bled and tested at day 28. This procedure is repeated at 2 week intervals until the desired antibody liters for both antigens are achieved.

### EXAMPLE 18

ELISA screening protocol: ELISA assays employ Costar® universal covalent surface immune assay plates and standard protocols for binding of the bacterial and host antigens. The system employs a two or three stage development system in which the adhesion molecule is covalently bound to the 96 well assay places by UV cross linking. The first stage anti-adhesin specific mouse serum or hybridoma supernatant antibodies is diluted and incubated in the plate wells after the adhesion molecules are bound and the plates blocked with BSA. After washing to remove non-specifically bound first stage reagents, a peroxidase coupled anti-mouse second stage antibody is added to the test wells, incubated, and washed. Substrate is then added to the wells to develop the assay which is subsequently read by an automated ELISA plate reader. If the desired sensitivity is not achieved, a third stage to amplify signals is employed.

### EXAMPLE 19

Hybridoma fusion protocol Anti-adhesion hybridomas are subcloned and selected as soon as hybridoma cell numbers permit. Subcloning is performed in 96-well plates by limiting dilution in HAT medium. Upon successful EL_{ISA} screening of mAb-positive subclones, the hybridomas are adapted to serum-free medium for bulk production of anti-adhesin mabs.

### EXAMPLE 20

Monoclonal antibodies (mAbs) directed against microbial adhesion molecules and host ligands can be used as diagnostic reagents and, in some instances, as therapeutic agents. For example, the inventors have shown that specific mAbs detect very low concentrations of shed microbial adhesion molecules (adhesins) in serum samples of mice infected with Candida albicans well before symptoms of the disease appear. In addition, the inventors have demonstrated the effectiveness of mAb directed against cell surface and verotoxin adhesive domains of verocytotoxic *Escherichia coli* to detect the organism or its toxin in meat samples.

### EXAMPLE 21

### Identification of peptide domains of adhesive epitopes

Epitope mapping using random phage-display libraries are used to determine key molecular structures embedded in the complex topologies of prokaryotic or eukaryotic cell membranes. Monoclonal antibodies prepared against purified glycoproteins or glycolipids or adhesin-positive microbes are used to probe a nonapeptide library (J404) displayed on a new vector M13KBst (Burritt, J.B. and C. W. Bond. J. Biochem. 238: 1-13). Affinity purification of phage bearing epitopes bound by mAbs is performed as follows: 1 x 10¹² phage from a phage nonapeptide display library is combined with 1.0 ml of Sepharose beads conjugated with 4 mg cf anti-adhesin blocking mAbs. The beads are mixed with the phage at 4°C for 16 hours by gentle inversion. The mixture is then loaded into a 5 ml plastic column barrel (Evergreen) and unbound phage removed by washing with 50 ml phage buffer (50 mM Tris HCl pH 7.5, 150 mM NaCl, 0.5% Tween 20 (v/v), 1 mg/ml BSA). Bound phage are eluded from the column with 2.0 ml of eluting buffer (0.1 M glycine, pH 2.2), and the pH of the eluate is neutralized immediately with four drops of 2 M Trizma base. The titer of phage (nonapeptide library) is determined for each column eluate by plaque assay according to standard procedures. The column matrices are preserved for reuse in second and third round affinity purification by washing with 10 ml PBS pH 7.0, followed by 3.0 ml PBS containing 0.02% sodium azide. The columns are stored at 4°C until the next affinity purification, and prepared for reuse by rinsing with 20 ml of phage buffer prior to mixing with amplified phage. As a control for antibody specific selection, one column is prepared without antibody bound to the beads; all steps of affinity purification of phage are carried out on this control column and a sample of the resulting phage is sequenced to provide data defining the peptide sequences recognized by adhesion blocking mAbs.

High affinity peptides able to block the adhesion of microorganisms to target cells *in vivo* can be used as therapeutic agents for the treatment or prevention of infectious diseases. These peptides can also be used as diagnostic reagents for dececting anti-adhesion antibodies produced by the host against PAMs.

### EXAMPLE 22

### Vaccine Preparation: Preparing DNA for insertion into live vector delivery systems.

DNA from affinity purified phage displaying adhesive peptides is extracted from infected K91 (*E. coli*) cells and purified on CsCl gradients. The adhesive peptide-encoded region of the phage DNA contains restriction sites to allow removal of a 31-base pair fragment from the vector DNA when cleaved with BstXI. Synthetic DNA inserts are then produced in accord with the method described by Cwila, S. et al (Proc. Nat. Acad. Sci. 87: 6378-6382). An asd stabilized plasmid carrying DNA encoding subunit adhesive domains is constructed and inserted into an attenuated *Salmonella typhimurium* strain (H683) as described by Wu, S et al (Infect. Immun. 63: 4933-4938). As an alternative *Salmonella typhic* or *shigella dysenterium* strain may also be engineered to express adhesive epitopes. The *Salmonella* vector displaying the adhesive peptides are administered orally and elicit powerful mucosal as well as systemic Th1 and Th2 immune responses.

An alternative method of deliver of antigens to inductive sites of immune compartments of the host exploits the anchor mechanism of the surface proteins of gram-positive bacteria (Fischetti, V. A. 1996. ASM News 62: 405-410) or intratracheal gene delivery with adenovirus vector (Van Ginkel, F. W. *et al* 1995. Hum. Gene. Ther. 6: 895-903).

The final product in the vertically integrated methodology of the inventors that detects, characterizes and replicates microbial adhesion molecules is a wide variety of subunit recombinant oral vaccines expressing or displaying peptide domains reflecting the topology of the microbial adhesion molecules. A live vector delivery system, for example described by Wu *et al* expressing the appropriate peptide epitopes can be constructed for every pathogenic microorganism that uses an adhesion mechanism to enter and infect the host.

It is clear from the present work that pathogenic organisms have acquired and use adhesion and signaling systems as a means of infecting the host by subverting or entering the cell-cell communications network. The similarities of host cell and invasive microbial trafficking events, including address recognition and signal transduction, imply that many microbial adhesion and signaling systems are evolutionary products of functional and molecular mimicry of host systems. The infectious trafficking cf pathogenic microbes involves at least two types of receptor-ligand interactions with target cells of the host-carbohydrate binding protein receptors and protein binding protein receptors. As with host cells, the first-order interaction of glycoprotein receptors with carbohydrate ligands serve to control the specificity of address recognition but not tight binding of the receptor/ligand pair. A second-order interaction involving protein receptors and protein/peptide ligands promotes tight binding and the augmentation of signal transduction events on the host cell membrane and/or in the cytosol. This principle has been demonstrated for neutrophil recruitment where recognition is mediated by protein (selectin)-carbohydrate (sialyl Le^{x}) binding but tight binding and flattening are the result of protein-protein (integrin/ligand) binding. Similarly, many bacteria and bacterial toxins including the Shiga-like toxin of enterohemorrhagic *E. coli* recognize a molecular address on endothelial cells through a protein receptor-carbohydrate ligand interaction and initiate signal transduction events via a protein-protein interaction that contributes to the disruption of intracellular functions (St. Hilare et al 1994. Biochem. 33: 14452-14463). The best characterized adhesion systems of pathogenic microbes are those of the protein receptor-carbohydrate ligand interactions between microbe and host cell (Mirelman, D. , and I. Ofek. 1996. In. Microbial Lectins and Agglutinins, p. 1-19. John Wiley and Sons. N.Y.). Many significant examples of microbial-host cell adhesion mediated by each type of receptor-ligand pairs are listed below.

| **Protein receptor-protein ligand interactions on the Cell Surface** | | | |
|---|---|---|---|
| *Bacterium* | *Virglence Protein* | Host homolog | Ligand |
| *B. pertussis* | Hemagglutinin | RGD site on fibrinogen | ICAM-1 |
| *Y enterolytica* | invasin | Host Cell (CD?) protein | *β1* integrin |
| *E. coli* | Intimin | Host cell (CD?) protein | β1 integrin |
| *Fungus* | | | |
| *C. albicans* | Adhesion | Integrin (LFA-1) | ICAM-1 |
| Leishmania *species* | adhesion | RGD site on fibronectin | Integrin(CR3) |

| **Protein receptor-protein ligand interactions on the Cell Surface** | | | |
|---|---|---|---|
| *Bactenum* | *Bacterial homolog* | Host homolog | Ligand |
| *H.* pylori | VacA toxin | Promotes vacuole formation | GTP-binding protein-Rab |
| *E. coli*/*Salmonella* | BGF-like protein | BGF-Promotes membrane ruffling and actin polymerization | GTP-binding protein-Ras |
| *C. botulinum* | C3 toxin | Controls actin rearrange | GTP-binding protein-Rho |
| *Listeria* | ActA protein | Induces actin polymerization | GTP-binding protein ? |

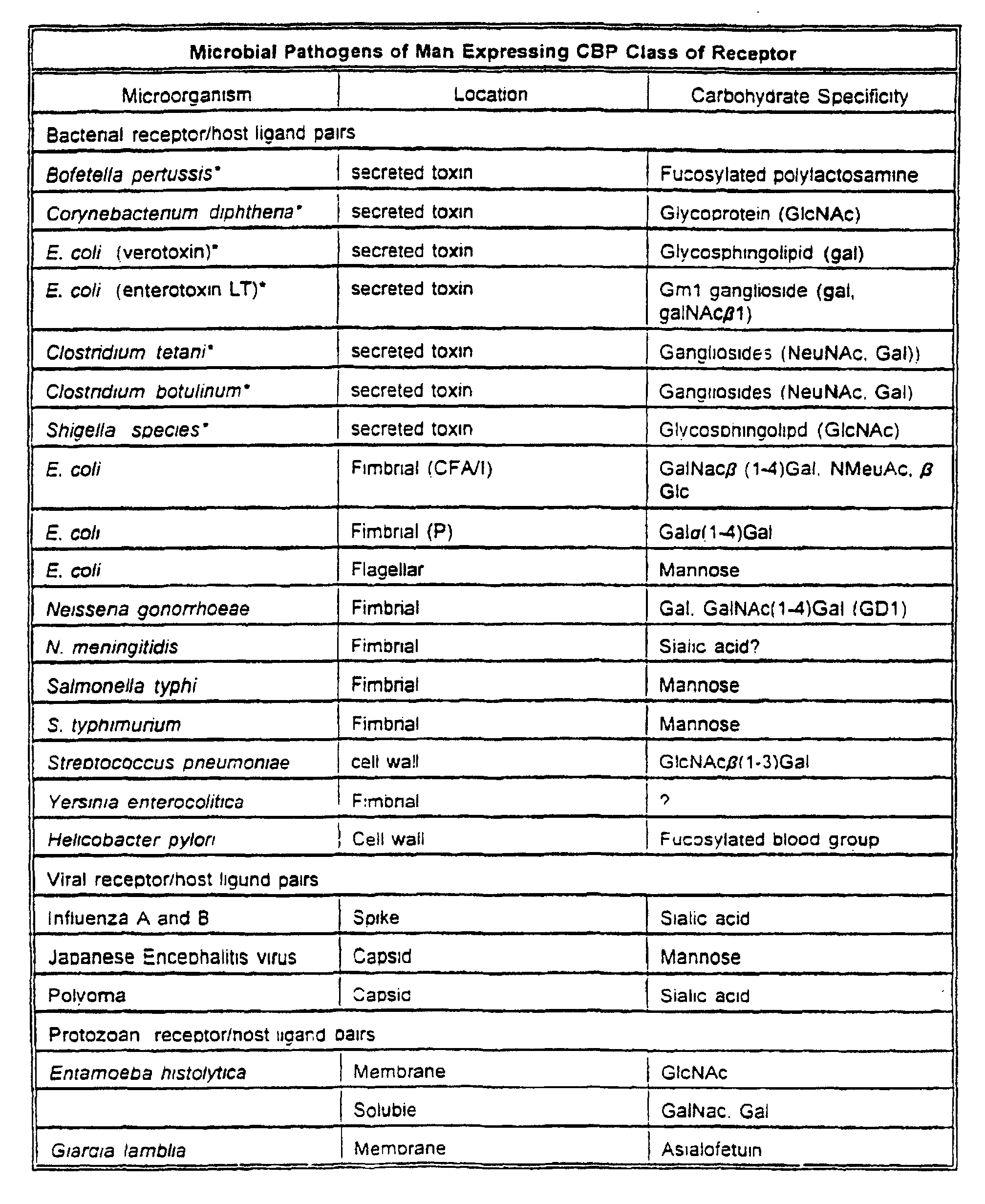

### The carbohydrate binding protein receptors

Many microbial attachment molecules, like the adhesion molecules (selectins) of inflammatory cell (leukocytes and endothelial cells) systems, are defined as lectin-like structures or carbohydrate binding proteins (CBP) that mediate adhesive interactions by recognition of carbohydrates on target cells (See: Microbial Lectins and Agglutinins. 1996. John Wiley and Sons, N.Y. and Jutila, M. A. *et al* 1989. In: Leukocyte Adhesion, Edited by Springer, *et al* Springer-Verlag, p. 211-219). These attachment molecules are chemically defined as glycoproteins and control a myriad of biological events. Microbial CBP receptors, like selectins on inflammatory cells, serve as molecules of recognition in cell-cell interactions. BCP receptors bind reversibly and noncovalently with mono or oligosaccharides, both simple and complex whether free in solution or on cell surfaces.

### The Protein binding Protein Receptors

An array of protein receptors used by microbes to bind to protein ligands can be found in all classes of pathogenic microorganisms. Typically, protein receptor and protein/peptide (PBP)) interactions promote tight binding of the pathogen to target cells of the host. Such binding elicits and/or augments the activation of signaling pathways that produce alterations in the actin network of eucaryotic cells. There appear to be two types or cellular sites of protein-protein adhesive interactions, the first occuring at the microbe-target cell interface and, the second, signal transduction events promoted by the interaction of proteins secreted by the pathogen with proteins of the actin network (the GTP-binding proteins -Rho, Rab, Arf, Ran, Ras, Rac, Cd-42, the MAPK cascade, and the t-SNARE, v-SNARE model for docking of vesicles) cf the host cell. In many of these systems, molecular mimicry or piracy of host adhesion molecules or their ligands by the pathogen has been documented as indicated in specific examples below.

### Microbial Pathogens of Man Expressing PBP Class of Receptors

Host adhesion molecules found in the integrin family and their protein/peptide ligands operating in cell-cell and cell-extracellular matrix interactions have been shown to be subverted by microbial homologs of integrins or their protein ligands. In the host, the *β1* integrins (the VLA family) bind to extracellular matrix components fibronectin, fibrinogen, laminin and collagen and they are expressed on many non-hematopoietic and leukocyte cells types. The β2 subfamily members are often referred to as the leukocyte integrins because their expression is limited to leukocytes. The LFA-1, Mac-1 and gp150.95 *β2* integrins are important in adhesion of myeloid cells to other cells and to ligands that become insolubilized during activation of the complement and clotting cascades. The structural domains of integrins have been correlated with ligand binding by crosslinking to peptides containing the sequence Arg-Gly-Asp (RGD), a ligand recognition motif for several but not all integrins. Molecular and functional mimicry of the adhesion moleclules of the host's integrin family and immunoglobulin superfamily have been observed (Hoepelman,, A. I. M. and E. I. Tuomanen. 1992.. Infect. Immunity. 60:1729-1733). Other pathogens exploiting mimicry of extracellular matrix proteins of the host display RGD sequences on their cell surfaces and, consequently, bind readily to integrins.

### Intracellular Protein-Protein Interactions

Pathogenic organisms have acquired an array of protein molecules that functionally mimic those involved in regulating the cytoskeleton of eucaryotic cells. These so-called virulence proteins interfere, for example, with a signaling cascade containing small guanosine triphosphate (GTP)-binding proteins (-Rho, Ras, Rac, Cdc42, etc.) that direct the function of the actin network of host cells. The virulence proteins appear to bind in a very specific manner to GTP-binding proteins and promote rearrangements of the actin network that benefit the microbe.

### Other Protein-Protein interactions

There are doubtlessly hundreds of distinct cytokines and dozens of cytokine-activated second-messenger systems that regulate immunity and inflammation. The interactions of cytokines with cells is largely a protein-protein/peptide adhesive event and many may be subverted or tapped into by microbial homologs.

Chemokines, originally classed as cytokines, are small pro-inflammatory peptides that are best known for their leukocyte-chemoattractant activity. The receptors, like their ligands, form a family of structurally and functionally related proteins. They are members of the superfamily of heptahelical, rhodopsin-like, G-protein-coupled receptors that can be defined by amino acid sequence homologies . The types A and B receptor for the leukocyte chemokine, IL-8, and the macrophage inflammatory protein 1α (MIP-1α)/RANTES receptor, are related by sequence and chemokine binding to two herpesvirus products.

The inventors develop recombinant subunit vaccines designed to be administered orally or intranasally. The oral vaccine is produced in accord with the method described by Wu et al., whereby a live vector *(Salmonella* *typhimurium)* system displaying or expressing the peptide epitopes on the surface of the organism is used to produce a potent mucosal and systemic immune response. Alternatively, another vector system co-developed by one of the inventors employs a aderiorovirus vector system for delivering critical epitopes to mucosal immune compartments of the upper respiratory tract. Similarly other viral, phage, bacterial or polymeric vectors may be used.

Mammalian selectins are not used as or in vaccines because they are solely of host origin and, therefore, non-immunogenic. Only peptide domains of microbial adhesive sites that bind carbohydrate ligands in the same fashion as selectins, or functional analogs thereof, are used for vaccine preparation. The microbial peptide domains incorporated into a vaccine formulation will be sufficiently antigenic to provoke an immune response.

Further, the vaccine and peptide compounds of the present invention are useful in pharmaceutical compositions for systemic administration to humans and animals in unit dosage forms, such as tablets, capsules, pills, powders, granules, suppositories, sterile parenteral solutions or suspensions, sterile non-parenteral solutions or suspensions oral solutions or suspensions, oil in water or water in oil emulsions and the like, containing suitable quantities of an active ingredient, as appropriate.

For oral administration either solid or fluid unit dosage forms can be prepared with the vaccine and peptides of the invention. The vaccine and peptides are useful in pharmaceutical compositions (wt%) of the active ingredient with a carrier or vehicle in the composition in about 1 to 20% and preferably about 5 to 15%.

Either fluid or solid unit dosage forms can be readily prepared for oral administration. For example, the vaccine can be mixed with conventional ingredients such as dicalciumphosphate, magnesium aluminum silicate, magnesium stearate, calcium sulfate, starch, talc, lactose, acacia, methyl cellulose and functionally similar materials as pharmaceutical excipients or carriers. A sustained release formulation may optionally be used. Capsules may be formulated by mixing the compound with a pharmaceutical diluent which is inert and inserting this mixture into a hard gelatin capsule having the appropriate size. If soft capsules are desired a slurry of the compound with an acceptable vegetable, light petroleum, or other inert oil can be encapsulated by machine into a gelatin capsule.

Suspensions, syrups and elixers may be used for oral administration of fluid unit dosage forms. A fluid preparation including oil may be used for oil soluble forms. A vegetable oil such as corn oil, peanut oil or safflower oil, for example, together with flavoring agents, sweeteners and any preservatives produces an acceptable fluid preparation. A surfactant may be added to water to form a syrup for fluid unit dosages. Hydroalcoholic pharmaceutical preparations may be used having an acceptable sweetener such as sugar, saccharine or a biological sweetener and a flavoring agent in the form of an elixer. Pharmaceutical compositions for parenteral and suppository administration can also be obtained using techniques standard in the art.

The above peptides or vaccine and other drugs can be present in the reservoir alone or in combination form with pharmaceutical carriers or adjuvants. The pharmaceutical carriers acceptable for the purpose of this invention are the art known carriers that do not adversely affect the drug, the host, or the material comprising the drug delivery device. Suitable pharmaceutical carriers include sterile water; saline, dextrose; dextrose in water or saline; condensation products of castor oil and ethylene oxide combining about 30 to about 35 moles of ethylene oxide per mole of castor oil; liquid acid; lower alkanols; oils such as corn oil; peanut oil, sesame oil and the like, with emulsifiers such as mono- or di-glyceride of a fatty acid, or a phosphatide, e.g., lecithin, and the like; glycols; polyalkylene glycols; aqueous media in the presence of a suspending agent, for example, sodium carboxymethylcellulose; sodium alginate; poly (vinyl-pyrolidone); and the like, alone, or with suitable dispensing agents such as lecithin; polyoxyethylene stearate; and the like. The carrier may also contain adjuvants such as preserving stabilizing, wetting, emulsifying agents and the like together with the penetration enhancer of this invention. Delivery systems for the vaccine also include phage (such as M13), a live vector, *Salmonella sp., Shigella sp.,* adenovirus, liposomes, cowpea mosaic virus, alginate gels, peptide conjugates, appropriate adjuvate systems and glycoconjugates. These delivery systems all have proteins or active molecules expressed on their surface.

The effective dosage for mammals may vary due to such factors as age, weight, activity level or condition of the subject being treated. Typically, an effective vaccinating dosage of a compound according to the present invention is about 10 to 500 mg when administered. Vaccination should be consistent with protocols set forth in *Remington's Pharmaceutical Sciences,* 18th Ed., Wiley Publications, 1990, incorporated herein by reference in its entirety.

### REFERENCES

1. Tuomanen, E. 1992. Subversion of leukocyte adhesion systems by respiratory pathogens. ASM News 59:292-296.
2. Falkow, S., 1991, Bacterial entry into eukaryotic cells. Cell 65:1099-1102.
3. Ishibashi, Y., Claus, S., and D.A. Relman, 1994. *Bordetella pertussis* filamentous hemagglutinin interacts with a leukocyte signal transduction complex and stimulates bacterial adherence to monocyte CR3 (CD11b/CD18). J. Exp. Med. 180:1225-1233.
4. Jutila, M.A., Lewinsohn, D.M. Berg., E.L. and E.C. Butcher 1989. Homing receptors in lymphocyte, monocyte and neutrophil interaction with endothelium. In: Leukocyte Adhesion, edited by Springer, et al. New York: Springer-Verlag, p. 211-219.
5. Lasky, L.A. 1992. Selectins: Interpreters of cell-specific carbohydrate information during inflammation. Science. 258:964-969.
6. Jutila, M.A. 1994. Selectins in leukocyte extravasation: function of a common epitope on L-and E-selectin. Adv. Pharmacol. 25:235-262.
7. Smith, C.W. 1993. Endothelial adhesion molecules and their role in inflammation. Can. J. Physiol. Pharmacol. 71:76-87.
8. Bevilacqua, M.P. and R.M. Nelson. 1993, Selectins. J. Clin. Invest. 91:379-387.
9. Wolber, F., R. Craig, O. Abassi, J. Ballew, R. Lobb and L. Stoolman, 1993. VLA-4 mediates lymphocyte binding to endothelium under shear. FASEB 7:A3704. (Abstract).
10. Berg, E.L., L.M. McEvoy, C. Berlin, R.F. Bargatze and E.C. Butcher. 1993. L-selectin mediated lymphocyte rolling on MAdCAM-1. Nature 366:695-698. venules. J. Exp. Med. 178:367-372.
11. Jutila, M. 1994. Selectins in leukocyte extravasation: function of a common epitope on L-and E-selectin. Adv. Pharmacol. 25:235-262.
12. Lawrence, M.B. and T.A. Springer. 1993. Neutrophils roll on E-selectin. J. Immunol. 151:6338-6346.
13. Smith, C.W. 1993. Endothelial adhesion molecules and their role in inflammation. Can. J. Physiol. Pharmacol. 71:76-87.
14. Lawrence, M.B. and T.A. Springer. 1991. Leukocytes roll on a selectin at physiologic flow rates: Distinction from and prerequisite for adhesion through integrins. Cell 65:859-873.
15. Butcher, E.C. 1986. The regulation of lymphocyte traffice. Curr. Top. Microbiol. Immunol. 128:85.
16. Ley, K.F. Gaehtgens, C. Fennie. M.S. Singer, L.A. Lasky and S.D. Rosen, 1991. Lectin-like adhesion molecule-1 mediates leukocyte rolling in mesenteric venules in vivo. Blood 77:2552-2555.
17. von Andrian, U.H., J.D. Chambers, E.L. Berg, S.A. Michie, D.A. Brown, D. Karolak, L. Ramezani, E.M. Berger, K.E. Arfors and E.C. Butcher. 1993. L-selectin mediates neutrophil rolling in inflamed venules through sialyl Lewis-dependent: and independent recognition pathways, Blood 82:182-191.
18. Abbassi, O., T.K. Kishomoto, C.L. Lane, L.V. McIntyre and C.W. Smith, 1993. Endothelial-leukocyte adhesion molecule-1 supports neutrophil rolling in vitro under conditions of flow. Submitted.
19. Bargatze, R., S. Kurk, G. Watts, T.K. Kishimoto, C.A. Speer, and M.A. Jutila. 1994. In vivo and in vitro functional examination of a conserved epitope of L- and E-selectin crucial for leukocyte-endothelial cell interactions. J. Immunol., 152:5814.
20. Ahuja, S., J.L. Gao and P.M. Murphy, 1994. Chemokine receptors and molecular mimicry. Immunol. Today. 15:281-287.
21. St. Hilaire, P.M., M.K. Boyd, and E.J. Toone. 1994. Interaction of the Shiga-like toxin Type 1 B-subunit with its carbohydrate receptor. Biochem. 33: 14452-14463.
22. Keusch, G.T., A. Donohue-Rolfe, and M. Jacewicz. 1986. Microbial lectins and agglutinins: Properties and biological activity. p. 271-295. John Wiley and sons, NY.
23. Cossar, P., P. Boquet, S. Normark, and R. Rappuoli. 1996. Cellular Microbiology Emerging. Science 271:315-316.
24. Baringa, M. 1996. A shared strategy for virulence. Science 272:1261-1263.
25. Ishibashi, U., Claus, S., and Relman, D.A. 1994. Bortella pertussis filamentous hemagglutinin interacts with a leukocyte signal transduction complex and stimulates bacterial adherence to monocyte CR3 (CD11b/CD18). J. Exp. Med. 180(4):1225.
26. Kimura, A., Mountzouros, K.T., Relman, D.A., Falkow, S., and Cowell, J.L. 1990. Bordetella pertussis filamentous hemagglutinin: evaluation as a protective antigen and colonizing factor in a mouse respiratory infection model. Infect. Immun. 58 (1) :7.
27. Falanga, P.B., and Butcher, E.C. 1991. Late treatment with anti-LFA-1 (CD11a) antibody prevents cerebral malaria in a mouse model. Eur. J. Immunol. 21:2259.
28. Ockenhcuse, C.F., et al. 1992. Human vascular endothelial cell adhesion receptors fcr Plasmodium falciparum-infected erythrocytes: Roles for endothelial leukocyte adhesion molecule 1 and vascular cell adhesion molecule 1. J. Exp. Med. 176:1183.
29. Cutler, J.E., D. L. Brawner, K.C. Hazen, and M.A. Jutila. 1990. Characteristics of *Candida albicans* adherence to mouse tissue. Infect. Immun. 58:1902-1908.
3C. Klotz, S.A., and Smith, R.L. 1991. A fibernectin receptor on *Candida albicans* mediates adherence of the fungus to extracellular matrix. JID. 163:604.
31. Relman, D., E. Tuomanen, S. Falkow, D.T. Golenbock, K. Saukonen, and S.D. Wright, 1990. Recognition of a bacterial adhesin by an eukaryotic integrin: CR3 on human macrophages binds filamentous hemagglutinin of *Bordetella pertussis.* Cell 61:1375-1382.
32. Saukonen, K., W.N. Burnette, V. kMar, H.R. Masure, and E. Tuomanen. 1992. Pertussis toxin has eukaryotic-like carbohydrate recognition domain. Proc. NMatl. Acad. Sci. 89:118-122.
33. van't Wout, J.W. N. Burnette, V. Mar, H. Sato, S.D. Wright and E. Tuomanen. 1992. Cooperative microbial adhesins: the B oligomer of pertussis toxin triggers enhanced FHA-mediated entry of Bordetella pertussis into human macrophages. Infect. Immun. 60:3303-3308.
34. Whittan, T.S., I.K. Wachsmuth, and R.A. Wilson. 1988. Genetic evidence of conal descent of *Escherichia coli* 0157:H7 associated with hemorrhagic colitis and hemolytic uremic syndrome. J. Infect. Dis. 157:1124-1133.
35. Karmalim, M.A., M. Petric, C. Lim, and J. Hockin. 1985. The associated between idiopathic hemolytic uremic syndrome and infection by vertoxin-producing Escherichia coli. J. Infect. Dis. 151:775-782.
36. Sherman, P. , R. Son i, M. Petric, and M. Karmali. 1987. Surface properties of the vero cytotoxin-producing Escherichia *coli* 0157:H7. Infect. Immun. 55:1825-1829. 37, Karch, H.J. Heesemann, R. Laufs, A.D. 0'Brier., C.O. Tacket, and MM. Levine 1987. A plasmid of enterohemorrhagic Escherichia *coli* 0157:H7 is required for expression of a new fimbrial antigen and for adhesion to epithelial cells. Infect. Immun. 55:455-461.
38. Dytoc., M. T., A. Ismaili, D. J. Philpott, R. Soni, J. L. Brunton, and P.M. Sherman. 1994. Distinct binding properties of eaeA-negative veroctytoxin producing *Escherichia coli* of serotype 0113:H21. Infect. Immun. 62: 3494-3505.
39. Dytoc, M. B. Gold, M. Louie, M. Huesca, L. Fedorko, S. Crow, C. Lingwood, J. Brunton and P. Sherman. 1993. Comparison of *Helicobacter pylori* and attaching-effacing Escherichia coli adhesion to eukaryotic cells in vitro. Infect. Immun. 61: 448-456.
40. Louie, M., J.D. Azavedo, R. Clarke, A. Borczyk, H. Lior, M. Richter and J. Brunton. 1994. Sequence heterogeneity of the eae gene and detection of verotoxin-producing Escherichia coli using a serotype-specific primners. Epidemiol. Infect. 112: 449-461.
41. Donnenberg, M.S. and J.B. Kaper. 1992. Enteropathogenic *Eshcerichia coli.* Infect. Immun. 60: 3953-3961.
42. Donnenberg, M.S., J.A. Nataro and J.B. Kaper. 1992. A plasmid-encoded type IV fimbrial gene of enteropathogenic. Escherichia *coli* associated with localized adherence. Mol. Microbic. 6: 3427-3437.
43. Yu, J., and J.B. Kaper. 1992. Cloning and characterization of the eae gene of enterohemorrhagic Escherichia coli 0157:H7. Mol. Microbiol. 6:411-417.
44. Tran Van ZNheiu, G., and R.R. Isberg. 1991. The *Yersinia pseudotub erculosis* invasin protein and human fibronectin bind to mutually exclusive sites on the alpha 5 betal integrin receptor. J. Biol. Chem. 266: 24367-24375.
45. Newell, D.G. 1991. Virulence factors of *Helicobacter* pylori. Scand. J. Gastroenterol. Suppl. 187:31-38.
46. Boren, T.S. Normark and P. Falk, 1994. *Helicobacter pylori:* molecular basis for host recognition and bacterial adherence. Trends Microbiol. 2:221-228.
47. Lindberg, AA. et al., 1987. Identification of the carbohydrate receptor for Shiga toxin produced by Shigella dysenterie type 1. Biochem. 262: 1779-1785.
48. Finne, J. 1985. Polysialic acid a glycoprotein carbohydrate involved in neural adhesion and bacterial meningitis. TIBS.
49. Tjia, K.F., J.P. Putten, E. Pels, and H.C. Zanen. The interaction between *Neisseria gonorrhoeae* and the human cornea in organ culture. Graefes Arch. Clin. Exp. Ophthalmol. 1988. 226:341-345.
50. Weel, J.F., C.T. Hopman and J.P. van Putten. In situ expression of *Neisseria gonorrhoeae* opacity proteins in infected epithelial cells; apparent role of Opa proteins in celluar invasion. J. Exp. Med. 1991. 174:705-715.
51. Soteriadou, K.P., M.S. Remoundos, M.C. Katsikas, A.K. Tzinia, V. Tsikaris, C. Sakarellos S.J. Tzartos. 1992. The Ser-arg-tyr-Asp region cf the major surface glycoprotein of *Leish* mimics the arg-gly-asp-ser cell attachment region of fibronectin. J. Biol. Chem. 267:1398-13985.
52. Patti, J.M., B.L. Allen, M. J. McGavin and M. Hook. 1994. MSCRAMM-Mediated Adherence of Microorganisms to Host Tissues. Annu. Rev. Micro. 48:585-616.
53. Kaplan, B.S., T.G. Cleary and T.G. Obrig. 1990. Recent advances in understanding the pathogenesis of the hemolytic uremic syndromes. Pediatr. Nephrol. 4:276-283.
54. Wu, S. , D. W. Pascual, J.L. VanCott, J.R. McGhee, D.R. Maneval, Jr., M.M. Levine, a M. Hone. 1995. Immune responses to novel *Escherichia coli and Salmonella typhimuriu* vectors that express colonization factor antigen I (CFA/1) of enterotoxigenic *E.* coli in the absence of the CFA/1 positive regulator cfaR. Infect. Immunity. 53:4933-4938.
55. Pascual. et al. 1996. Mucosal Immunity: molecular and cellular aspects of immune protection to enteric infections in: Enteric infections and immune responses. Eds. Paradise, Bendinelli and Friedman. p. 15-35. Plenum Press, NY.
56. Briggs, J.B., Y. Oda, J.H. Gilbert, M.E. Schaefer, and B.A. Macher. 1995. Peptides inhibit selectin-mediated adhesion in vitro and neutrophil influx into inflammatory sites in vivo. Glycobiol. 5(6): 583-588.
57. Spevak, W., C. Foxall, D. H. Charych, F. Dasgupta, and J.O. Nagy. 1996. Carbohydrates in acidic multivalent assembly: Nanomolar P-selectin inhibitors. J. Med. Chem. 39: 1018-
58. Burritt, J. B., and C. W. Bond. 1996. Filamentous phage display of oligopeptide libraries. A Biochem. 238:1-13.

The purpose of the above description and examples is to illustrate some embodiments of the present invention without implying any limitation. It will be apparent to those of skill in the art that various modifications and variations may be made to the composition and method of the present invention without departing from the spirit or scope of the invention. All patents and publications cited herein are incorporated by reference in their entireties.

## Claims

1. A vaccine comprising a pharmaceutically acceptable carrier and an isolated pathogen adhesin molecule or immunogenic fragment thereof, wherein the pathogen adhesin molecule or immunogenic fragment thereof specifically binds to an adhesion molecule on a host cell or extracellular matrix thereby enabling said pathogen to traffic through host tissue, and wherein said vaccine induces a therapeutically effective immune response against the pathogen.

2. The vaccine of claim 1, wherein said pathogen adhesin molecule or immunogenic fragment thereof specifically binds to said adhesion molecule on a host cell or extracellular matrix under shear conditions *in vitro.*

3. The vaccine of claim 3, wherein said shear conditions are selected from the group consisting of physiological shear conditions as characteristically found in the: (1) vascular system; (2) respiratory system; (3) gastrointestinal tract; and (4) urinary tract.

4. The vaccine of claim 1, wherein said host cell is selected from the group consisting of leukocytes, endothelial cells, epithelial cells and cells of the nervous system.

5. The vaccine of claim 1, wherein said pathogen adhesin molecule functionally mimics a ligand for said host adhesion molecule.

6. The vaccine of claim 1, wherein said host adhesion molecule is a receptor for a host ligand selected from the group consisting of C-type lectins, selectins, integrins, members of the immunoglobulin superfamily and cytokines.

7. The vaccine of claim 6, wherein said selectins are selected from the group consisting of E-selectin, L-selectin and P-selectin.

8. The vaccine of claim 6, wherein said integrin is selected from the group consisting of VLA-1, VLA-2, VLA-3, VLA-4, VLA-5, VLA-6, leucam, Mac-1, LFA-1, gp150.95, CD41a, CD49 and CD51.

9. The vaccine of claim 6, wherein said member of the immunoglobulin superfamily is selected from the group consisting of ICAM-1, ICAM-2 or ICAM-3, VCAM, NCAM and PECAM.

10. The vaccine of claim 1, wherein said host adhesion molecule is selected from the group consisting of proteins, glycoproteins, glycolipids and carbohydrates.

11. The vaccine of claim 1, wherein said pathogen adhesin molecule binds to a carbohydrate ligand selected from the group consisting of residues of N-acetylneuraminic acid, sialic acid, N-acetylglucosamine, N-acetylgalactosamine, glucosamine, galactosamine, galactose, mannose, fucose and lactose.

12. The vaccine of claim 1, wherein said host adhesion molecule is a guanosine triphosphate-binding protein.

13. The vaccine of claim 1, but wherein said guanosine triphosphate-binding protein is selected from the group consisting of Rho, Ras, Rac, Dcd42, Rab, Ran and Arf.

14. The vaccine of claim 1, wherein said host cell is selected from the group consisting of cytokine-stimulated endothelial cells and endothelial cells expressing ICAM-1, CAM-1, MAdCAM-1 and PNAd-1.

15. The vaccine of claim 1, wherein said pathogen is selected from the group consisting of viruses, bacteria, protozoa and fungi.

16. The vaccine of claim 15, wherein said pathogen is a virus.

17. The vaccine of claim 15, wherein said pathogen is a bacterium.

18. The vaccine of claim 15, wherein said pathogen is a protozoa.

19. The vaccine of claim 15, wherein said pathogen is a fungus.

20. The vaccine of claim 15, wherein the pathogen is an intestinal tract pathogen selected from the group consisting of *Vibrio cholerae,* uropathogenic *Escherichia coli,* enterohemorrhagic *E. coli,* enteropathogenic *E. coli,* Salmonella species, *Shigella* species, *Pseudomonas* species, *Proteus* species, *Klebsiella pneumoniae, Aerobacter areogenes,* and *Heliobacter pylori.*

21. The vaccine of claim 15, wherein said pathogen is selected from the blood cell group consisting of *Plasmodium berghei, Plasmodium falciparum, Brucella species, Neisseia meningitidis, Staphylococcus species, Pasteurella pestis, Leishmania, Trypanosoma* and *Pasteurella tularensis.*

22. The vaccine of claim 15, wherein said pathogen is selected from the group consisting of *Mycobacterium tuberculosis, Legionella, Staphylococcus species, Streptococcus species, Bordetella pertussis, Pasteurella pestis, Hemophilus influenzae, and Cprumebacterum diphtheriae.*

23. The vaccine of claim 15, wherein said pathogen is selected from the fungal parasite group consisting of *Blastomyces, Aspergillus, Cryptococcus, Candida, Histoplasma, Coccidioides and Phycomycetes.*

24. The vaccine of claim 15, wherein said pathogen is selected from the intestinal parasite group consisting of *Entamoeba histolytica, Giardia lamblia, and Cryptosporidium.*

25. The vaccine of claim 15, wherein said pathogen is selected from the genito-urinary tract group consisting of *Neisseria gonorrhoeae, Chlamydia, Treponema pallidium, Trichomonas vaginales,* and *Tritrichomonas foetus.*

26. The vaccine of claim 15, wherein said pathogen is selected from the virus group consisting of *Influenza A, Influenza B, Influenza C, Measles virus, Mumps virus, Adenovirus, Rhinovirus, Poliovirus, Hepatitis virus, Hantavirus, Herpesvirus, Rubella, Human Immunodeficiency virus (HIV) and Coxsackieviruses.*

27. The vaccine of claim 1, wherein said host cell is selected from the group of respiratory system cells consisting of alveolar macrophages and endothelial and epithelial cells of the nasopharynx and alveoli.

28. The vaccine of claim 1, wherein said vaccine further comprises peptide domains of the adhesive region on the ∃-oligomer of an exotoxin selected from a group consisting of *Corynebacterium diptheriae exotoxin, Bordetella pertussis* toxin, *Shigella dysenteriae* (Type I) toxin, Salmonella *typhimurium* toxin, *Vibrio cholerae* toxin, enterhemorrhagic *Escherichia coli verotoxin,* Enteropathogenic *Escherichia coli enterotoxin, Pseudomonas* aeruginosa *exotoxin, Clostriudium tetani exotoxin and Clostridium botulinum exotoxin.*

29. The vaccine of claim 1, wherein said vaccine further comprises peptide domains of the adhesive lectin region on fimbriae displayed on microbes selected from a group consisting of *Escherichia coli, Neisseria gonorrhoeae, Neisseria meningitidis, Salmonella typhi, Salmonella tyhpimurim, other Salmonella species, Pseudomonas aeruginosa and Yersinia enterocolitica.*

30. The vaccine of claim 1, wherein said vaccine further comprises peptide domains of glycoprotein adhesion molecules on the cell surface of microbes selected from a group consisting of *Escherichia coli, Yersinia pseudotuberculosis, Helicobacter pylori, Vibrio cholera, Salmonella typhi, Salmonella typhimurium, Shigella dysenteriae, Leishmania, Giardia lambia, Entamoeba histolytica, Candida albicans and Harnia alvae.*

31. The vaccine of claim 1, wherein said vaccine further comprises peptide domain of glycoprotein adhesion molecules that bind to sialic acid ligands on nervous systems cells.

32. The vaccine of claim 1, wherein said vaccine comprises peptide domains of a microbial glycoprotein adhesion molecule selected from a group consisting of *Neisseria meningitidis* and *Escherichia coli* K1.

33. The vaccine of claim 1, further comprising a delivery system displaying said pathogen adhesion molecule or immunogenic fragment thereof.

34. The vaccine of claim 33, where said delivery system is selected from the group consisting of phage, a live vector, liposomes, M13 phage, cowpea mosaic virus, alginate gels, peptide conjugates, and glycoconjugates.

35. The vaccine of claim 34, wherein said live vector is a *Salmonella* species, a *Shigella* species or adenovirus.

36. A vaccine comprising a pharmaceutically acceptable carrier and an isolated pathogen adhesin molecule or immunogenic fragment thereof, wherein the pathogen adhesin molecule specifically binds to an adhesion molecule on a host cell or extracellular matrix under physiologic shear conditions *in vitro,* and wherein said vaccine induces a therapeutically effective immune response against the pathogen.

37. A vaccine comprising a pharmaceutically acceptable carrier and an isolated pathogen adhesin molecule mimetic, wherein said mimetic induces a therapeutically effective immune response against the pathogen.

38. A method of obtaining a vaccine against a pathogen comprising the steps of:
(a) selecting a pathogen adhesin molecule or fragments thereof that specifically binds to an adhesion molecule on a host cell or extracellular matrix;
(b) developing one or more monoclonal antibodies (mAbs) directed against at least one region of the isolated adhesin molecule or fragments thereof;
(c) isolating epitopes bound by said mAbs; and
(d) screening said selected epitopes to identify a molecule that induces a therapeutically effective immune response against the pathogen.

39. The method of claim 38, wherein said step of selecting is performed by a shear assay using target cells that express the host adhesion molecule.

40. The method of claim 38, further comprising a step of analyzing the specificity and blocking properties of mAbs for the pathogen/host cell interactions by a shear assay.

41. The method of claim 38, wherein said step of isolating epitopes includes screening a phage display library to identify mimetics of the pathogen adhesin molecule that also bind to said one or more monoclonal antibodies.

42. A diagnostic assay kit, comprising a monoclonal antibody specific for a pathogen adhesin molecule, wherein said molecule is capable of binding specifically to an adhesion molecule on a host cell or extracellular matrix under shear conditions in *vitro.*

43. The diagnostic assay kit of claim 42, wherein said pathogen adhesin molecule functionally mimics a ligand for said host adhesion molecule.

44. The diagnostic assay kit of claim 42, further comprising superparamagentic beads coated with said monoclonal antibodies.

45. A diagnostic assay kit, comprising a peptide or oligopeptide that functionally mimics the adhesive domain of a pathogen adhesin molecule, wherein said molecule specifically binds to an adhesion molecule on a host cell or extracellular matrix under shear conditions *in vitro.*

46. A diagnostic assay test composition comprising a glycolipid matrix displaying host adhesion molecules presenting a residue selected from the group consisting of residues of N-acetylneuraminic acid, sialic acid, N-acetylglucosamine, N-acetylgalactosamine, glucosamine, galactosamine, galactose, mannose, fucose and lactose; wherein said host adhesion molecule binds specifically to a pathogen adhesin molecule under shear conditions *in vitro.*

47. A therapeutic composition comprising a pharmaceutically acceptable carrier and an isolated pathogen adhesin molecule or fragment thereof, wherein said pathogen adhesin molecule or fragment specifically binds to an adhesion molecule on a host cell or extracellular matrix under shear conditions *in vitro.*

48. The therapeutic composition of claim 46, wherein said composition inhibits one or more of the following events associated with infection by the pathogen of an infected host, said events being selected from the group consisting of: (1) recognition by the pathogen of specific host cells or extracellular matrix; (2) shear dependent attachment of the pathogen to host cells or extracellular matrix; (3) activation dependent adhesion of the pathogen; (4) signal transduction mediated by the pathogen; (5) transendothelial migration of the pathogen; (6) passage by the pathogen through epithelia; (7) colonization by the pathogen; and (8) binding of a toxin produced by the pathogen to host cells or extracellular matrix.

49. The therapeutic composition of claim 48, wherein said composition binds to members of the selectin family of host adhesion molecules.

50. The therapeutic composition of claim 48, wherein said composition binds to members of the immunoglobulin superfamily of host adhesion molecules.

51. The therapeutic composition of claim 48, wherein said composition binds to members of the integrin family of host adhesion molecules.

52. The therapeutic composition of claim 48, wherein said pathogen adhesin molecule functionally mimics a ligand for said host adhesion molecule.

53. The therapeutic composition of claim 52, wherein the host adhesion molecule is a receptor for a host ligand selected from the group consisting of selectins, integrins, members of the immunoglobulin superfamily, cytokines and guanosine triphosphate-binding proteins.

54. The therapeutic composition of claim 53, wherein said selectin is selected from the group consisting of E-selectin, L-selectin and P-selectin.

55. The therapeutic composition of claim 53, wherein said integrin is selected from the group consisting ofVLA-1, VLA-2, VLA-3, VLA-4, VLA-5, VLA-6, leucam, Mac-1, LFA-1, gp150.95, CD41a, CD49 and CD51.

56. The therapeutic composition of claim 53, wherein said member of the immunoglobulin superfamily is selected from the group consisting of ICAM-1, ICAM-2 or ICAM-3, VCAM, NCAM and PECAM.

57. The therapeutic composition of claim 53, wherein said host adhesion molecule is a guanosine triphosphate-binding protein.

58. The therapeutic composition of claim 57, wherein said guanosine triphosphate-binding protein is selected from the group consisting of Rho, Ras, Rac, Dcd42, Rab, Ran and Arf.

59. The vaccine of claim 1 formulated for mucosal delivery, wherein the vaccine is administered orally or intranasally.

60. The vaccine of claim 1, wherein said pathogen adhesin molecule is selected from the group consisting of proteins, glycoproteins, glycolipids, carbohydrates, lectins, selectins, integrins and functional equivalents thereof.

## Patentansprüche

1. Vaccin, umfassend einen pharmazeutisch annehmbaren Träger und ein isoliertes pathogenes Adhesinmolekül oder ein immunogenes Fragment davon, wobei das pathogene Adhesinmolekül oder das immunologische Fragment davon spezifisch an ein Adhäsionsmolekül einer Wirtszelle oder einer extrazellulären Matrix bindet, was es dem Pathogen erlaubt, durch Wirtsgewebe zu gehen, und wobei das Vaccin eine therapeutisch wirksame Immunantwort gegen das Pathogen hervorruft.

2. Vaccin gemäss Anspruch 1, wobei das pathogene Adhesinmolekül oder das immunogene Fragment davon in vitro spezifisch an das Adhäsionsmolekül einer Wirtszelle oder extrazellulären Matrix unter Scherbedingungen bindet.

3. Vaccin gemäss Anspruch 3, wobei die Scherbedingungen ausgewählt sind aus der Gruppe, bestehend aus physiologischen Scherbedingungen, wie sie charakteristisch gefunden werden in: (1) vaskulären Systemen; (2) dem Atemsystem; (3) dem Gastrointestinaltrakt und (4) dem Harnapparat.

4. Vaccin gemäss Anspruch 1, wobei die Wirtszelle ausgewählt ist aus der Gruppe, bestehend aus Leukozyten, Endothelzellen, Epithelzellen und Zellen des Nervensystems.

5. Vaccin gemäss Anspruch 1, wobei das pathogene Adhesinmolekül funktionell einen Liganden für das Wirtsadhäsionsmolekül imitiert.

6. Vaccin gemäss Anspruch 1, wobei das Wirtsadhäsionsmolekül ein Rezeptor für einen Wirtszellenliganden ist, ausgewählt aus der Gruppe, bestehend aus Lectinen vom C-Typ, Selectinen, Integrinen, Mitgliedern der Immunoglobulin-Superfamilie und Cytokinen.

7. Vaccin gemäss Anspruch 6, wobei die Selectine ausgewählt sind aus der Gruppe, bestehend aus E-Selectin, L-Selectin und P-Selectin.

8. Vaccin gemäss Anspruch 6, wobei das Integrin ausgewählt ist aus der Gruppe, bestehend aus VLA-1, VLA-2, VLA-3, VLA-4, VLA-5, VLA-6, Leucam, Mac-1, LFA-1, gp150.95, CD41a, CD49 und CD51.

9. Vaccin gemäss Anspruch 6, wobei das Mitglied der Immunoglobulin-Superfamilie ausgewählt ist aus der Gruppe, bestehend aus ICAM-1, ICAM-2 oder ICAM-3, VCAM, NCAM und PECAM.

10. Vaccin gemäss Anspruch 1, wobei das Wirtsadhäsionsmolekül ausgewählt ist aus der Gruppe, bestehend aus Proteinen, Glycoproteinen, Glycolipiden und Kohlenhydraten.

11. Vaccin gemäss Anspruch 1, wobei das pathogene Adhesinmolekül an einen Kohlenhydratliganden, ausgewählt aus der Gruppe, bestehend aus Resten von N-Acetylneuraminsäure, Sialinsäure, N-Acetylglucosamin, N-Acetylgalactosamin, Glucosamin, Galactosamin, Galactose, Mannose, Fucose und Lactose, bindet.

12. Vaccin gemäss Anspruch 1, wobei das Wirtsadhäsionsmolekül ein Guanosintriphosphat-Bindungsprotein ist.

13. Vaccin gemäss Anspruch 1, wobei das Guanosintriphosphat-Bindungsprotein ausgewählt ist aus der Gruppe, bestehend aus Rho, Ras, Rac, Dcd42, Rab, Ran und Arf.

14. Vaccin gemäss Anspruch 1, wobei die Wirtszelle ausgewählt ist aus der Gruppe, bestehend aus Cytokinstimulierten Endothelzellen und Epithelzellen, die ICAM-1, CAM-1, MAdCAM-1 und PNAd-1 exprimieren.

15. Vaccin gemäss Anspruch 1, wobei das Pathogen ausgewählt ist aus der Gruppe, bestehend aus Viren, Bakterien, Protozoen und Pilzen.

16. Vaccin gemäss Anspruch 15, wobei das Pathogen ein Virus ist.

17. Vaccin gemäss Anspruch 15, wobei das Pathogen ein Bakterium ist.

18. Vaccin gemäss Anspruch 15, wobei das Pathogen ein Protozoon ist.

19. Vaccin gemäss Anspruch 15, wobei das Pathogen ein Pilz ist.

20. Vaccin gemäss Anspruch 15, wobei das Pathogen ein Pathogen des Verdauungstrakts ist, ausgewählt aus der Gruppe, bestehend aus Vibrio cholerae, harnpathogenen Escherichia coli, enterohämorrhagischen E. coli, enteropathogenen E. coli, Salmonellaarten, Shigellaarten, Pseudomonasarten, Proteusarten, Klebsiella pneumoniae, Aerobacter areogenes und Heliobacter pylori.

21. Vaccin gemäss Anspruch 15, wobei das Pathogen ausgewählt ist aus der Blutzellengruppe, bestehend aus Plasmodium berghei, Plasmodium falciparum, Brucellaarten, Neisseria meningitidis, Staphylococcusarten, Pasteurella pestis, Leishmania, Trypanosoma und Pasteurella tularensis.

22. Vaccin gemäss Anspruch 15, wobei das Pathogen ausgewählt ist aus der Gruppe, bestehend aus Mycobacterium tuberculosis, Legionella, Staphylococcusarten, Streptococcusarten, Bordetella pertussis, Pasteurella pestis, Hemophilus influenzae und Cprumebacterum diphtheriae.

23. Vaccin gemäss Anspruch 15, wobei das Pathogen ausgewählt ist aus der Pilzparasitengruppe, bestehend aus Blastomyces, Aspergillus, Cryptococcus, Candida, Histoplasma, Coccidioides und Phycomycetes.

24. Vaccin gemäss Anspruch 15, wobei das Pathogen ausgewählt ist aus der Darmparasitengruppe, bestehend aus Entamoeba histolytica, Giardia lamblia und Cryptosporidium.

25. Vaccin gemäss Anspruch 15, wobei das Pathogen ausgewählt ist aus der Genital-Harnapparat-Gruppe, bestehend aus Neisseria gonorrhoeae, Chlamydia, Treponema pallidium, Trichomonas vaginales und Tritrichomonas foetus.

26. Vaccin gemäss Anspruch 15, wobei das Pathogen ausgewählt ist aus der Virusgruppe, bestehend aus Influenza A, Influenza B, Influenza C, Measlesvirus, Mumpsvirus, Adenovirus, Rhinovirus, Poliovirus, Hepatitisvirus, Hantavirus, Herpesvirus, Rubella, Human-Immundefizienzvirus (HIV) und Coxsackieviren.

27. Vaccin gemäss Anspruch 1, wobei die Wirtszelle ausgewählt ist aus der Gruppe, bestehend aus Zellen des Atemwegsystems, bestehend aus Alveolarmacrophagen und Endothel- und Epithelzellen des Nasen-Rachen-Raums und Alveolen.

28. Vaccin gemäss Anspruch 1, wobei das Vaccin weiterhin Peptiddomänen des adhäsiven Bereichs des ∃-Oligomers eines Exotoxins, ausgewählt aus der Gruppe, bestehend aus Corynebacterium diphtheriae-Exotoxin, Bordetella pertussis-Toxin, Shigella dysenteriae (Typ I)-Toxin, Salmonella typhimurium-Toxin, Vibrio cholerae-Toxin, enterhämorrhagischem Escherichia coli-Verotoxin, enteropathogenem Escherichia coli-Enterotoxin, Pseudomonas aeruginosa-Exotoxin, Clostridium tetani-Exotoxin und Clostridium botulinum-Exotoxin, umfasst.

29. Vaccin gemäss Anspruch 1, wobei das Vaccin weiterhin Peptiddomänen des adhäsiven Lectinbereichs von Fimbrien bei Mikroben, ausgewählt aus der Gruppe, bestehend aus Escherichia coli, Neisseria gonorrhoeae, Neisseria meningitidis, Salmonella typhi, Salmonella typhimurium, andere Salmonellaarten, Pseudomonas aeruginosa und Yersinia enterocolitica, umfasst.

30. Vaccin gemäss Anspruch 1, wobei das Vaccin weiterhin Peptiddomänen der Glycoprotein-Adhäsionsmoleküle auf der Zelloberfläche von Mikroben, ausgewählt aus der Gruppe, bestehend aus Escherichia coli, Yersinia pseudotuberculosis, Heliobacter pylori, Vibrio cholera, Salmonella typhi, Salmonella typhimurium, Shigella dysenteriae, Leishmania, Giardia lambia, Entamoeba histolytica, Candida albicans und Harnia alvae, umfasst.

31. Vaccin gemäss Anspruch 1, wobei das Vaccin weiterhin eine Peptiddomäne der Glycoprotein-Adhäsionsmoleküle, die Sialinsäureliganden auf Zellen des Nervensystems binden, umfasst.

32. Vaccin gemäss Anspruch 1, wobei das Vaccin Peptiddomänen eines mikrobiellen Glycoprotein-Adhäsionsmoleküls, ausgewählt aus der Gruppe, bestehend aus Neisseria meningitidis und Escherichia coli K1, umfasst.

33. Vaccin gemäss Anspruch 1, weiterhin umfassend ein Zuführsystem, das das pathogene Adhäsionsmolekül oder das immunogene Fragment davon aufzeigt.

34. Vaccin gemäss Anspruch 33, wobei das Zuführsystem ausgewählt ist aus der Gruppe, bestehend aus einem Phagen, einem Lebendvektor, Liposomen, M13-Phagen, Augenbohne-Mosaikvirus, Alginatgelen, Peptidkonjugaten und Glycokonjugaten.

35. Vaccin gemäss Anspruch 34, wobei der Lebendvektor eine Salmonellenart, eine Shigellaart oder Adenovirus ist.

36. Vaccin, umfassend einen pharmazeutisch annehmbaren Träger und ein isoliertes pathogenes Adhesinmolekül oder ein immunogenes Fragment davon, wobei das pathogene Adhesinmolekül spezifisch an ein Adhäsionsmolekül einer Wirtszelle oder an extrazelluläre Matrix bei physiologischen Scherbedingungen in vitro bindet und wobei das Vaccin eine therapeutisch wirksame Immunantwort gegen das Pathogen hervorruft.

37. Vaccin, umfassend einen pharmazeutisch annehmbaren Träger und einen isolierten pathogenen Adhesin-Molekülimitator, wobei dieser Imitator eine therapeutisch wirksame Immunantwort gegen das Pathogen hervorruft.

38. Verfahren zum Erhalt eines Vaccins gegen ein Pathogen, umfassend die Schritte:
(a) Auswählen eines pathogenen Adhesinmoleküls oder eines Fragments davon, das spezifisch an ein Adhäsionsmolekül auf einer Wirtszelle oder der extrazellulären Matrix bindet;
(b) Entwickeln eines oder mehrerer monoklonaler Antikörper (mAbs), der/die gegen mindestens einen Bereich des isolierten Adhesinmoleküls oder Fragments davon gerichtet ist/sind;
(c) Isolieren der Epitope, die durch diese mAbs gebunden sind; und
(d) Screenen der ausgewählten Epitope, um ein Molekül zu identifizieren, das eine therapeutisch wirksame Immunantwort gegen das Pathogen induziert.

39. Verfahren gemäss Anspruch 38, wobei der Schritt des Auswählens durchgeführt wird durch einen Scherassay unter Verwendung von Zielzellen, die das Wirtsadhäsionsmolekül exprimieren.

40. Verfahren gemäss Anspruch 38, weiterhin umfassend einen Schritt des Analysierens der Spezifität und der Blockierungseigenschaften der mAbs auf die Pathogen/Wirtszell-Interaktion mittels eines Scherassays.

41. Verfahren gemäss Anspruch 38, wobei der Schritt des Isolierens der Epitope das Screenen einer Phagendisplaybibliothek einschliesst, um Imitatoren des Pathogenadhesinmoleküls zu identifizieren, die ebenfalls an den/die einen oder mehreren monoklonalen Antikörper binden.

42. Kit für einen diagnostischen Assay, umfassend einen für ein pathogenes Adhesinmolekül spezifischen monoklonalen Antikörper, wobei das Molekül in der Lage ist, spezifisch an ein Adhäsionsmolekül einer Wirtszelle oder der extrazellulären Matrix unter Scherbedingungen in vitro zu binden.

43. Kit für einen diagnostischen Assay gemäss Anspruch 42, wobei das pathogene Adhesinmolekül funktionell einen Liganden für das Wirtsadhäsionsmolekül imitiert.

44. Kit für einen diagnostischen Assay gemäss Anspruch 42, der weiterhin superparamagnetische Kügelchen, die mit diesen monoklonalen Antikörpern beschichtet sind, umfasst.

45. Kit für einen diagnostischen Assay, umfassend ein Peptid oder Oligopeptid, das funktionell die Adhäsionsdomäne eines pathogenen Adhesinmoleküls imitiert, wobei das Molekül spezifisch ein Adhäsionsmolekül auf einer Wirtszelle oder der extrazellulären Matrix unter Scherbedingungen in vitro bindet.

46. Testzusammensetzung für einen diagnostischen Assay, umfassend eine Glycolipidmatrix, die Wirtsadhäsionsmoleküle aufzeigt, die einen Rest, ausgewählt aus der Gruppe, bestehend aus Resten von N-Acetylneuraminsäure, Sialinsäure, N-Acetylglucosamin, N-Acetylgalactosamin, Glucosamin, Galactosamin, Galactose, Mannose, Fucose und Lactose, präsentiert; wobei das Wirtsadhäsionsmolekül spezifisch an ein pathogenes Adhesinmolekül unter Scherbedingungen in vitro bindet.

47. Therapeutische Zusammensetzung, umfassend einen pharmazeutisch annehmbaren Träger und ein isoliertes pathogenes Adhesinmolekül oder ein Fragment davon, wobei das pathogene Adhesinmolekül oder ein Fragment davon in vitro spezifisch an ein Adhäsionsmolekül auf einer Wirtszelle oder der extrazellulären Matrix unter Scherbedingungen bindet.

48. Therapeutische Zusammensetzung gemäss Anspruch 47, wobei die Zusammensetzung eines oder mehrere der folgenden Ereignisse, assoziiert mit einer Infektion eines infizierten Wirts durch das Pathogen, hemmt, wobei diese Ereignisse ausgewählt sind aus der Gruppe, bestehend aus: (1) Erkennen von spezifischen Wirtszellen oder von extrazellulärer Matrix durch das Pathogen; (2) scherabhängiges Anheften des Pathogens an Wirtszellen oder extrazelluläre Matrix; (3) aktivierungsabhängige Adhäsion des Pathogens; (4) durch das Pathogen vermittelte Signaltransduktion; (5) transendotheliale Migration des Pathogens; (6) Durchgang des Pathogens durch das Epithel; (7) Kolonisierung des Pathogens und (8) Binden eines durch das Pathogen hergestellten Toxins an Wirtszellen oder extrazelluläre Matrix.

49. Therapeutische Zusammensetzung gemäss Anspruch 48, wobei die Zusammensetzung an Mitglieder der Selectinfamilie der Wirtsadhäsionsmoleküle bindet.

50. Therapeutische Zusammensetzung gemäss Anspruch 48, wobei die Zusammensetzung an Mitglieder der Immunoglobulin-Superfamilie der Wirtsadhäsionsmoleküle bindet.

51. Therapeutische Zusammensetzung gemäss Anspruch 48, wobei die Zusammensetzung an Mitglieder der Integrinfamilie der Wirtsadhäsionsmoleküle bindet.

52. Therapeutische Zusammensetzung gemäss Anspruch 48, wobei das pathogene Adhesinmolekül funktionell einen Liganden für das Wirtsadhäsionsmolekül imitiert.

53. Therapeutische Zusammensetzung gemäss Anspruch 52, wobei das Wirtsadhäsionsmolekül ein Rezeptor für einen Wirtsliganden, ausgewählt aus der Gruppe, bestehend aus Selectinen, Integrinen, Mitgliedern der Immunoglobulin-Superfamilie, Cytokinen und Guanosintriphosphat-Bindungsproteinen, ist.

54. Therapeutische Zusammensetzung gemäss Anspruch 53, wobei das Selectin ausgewählt ist aus der Gruppe, bestehend aus E-Selectin, L-Selectin und P-Selectin.

55. Therapeutische Zusammensetzung gemäss Anspruch 53, wobei das Integrin ausgewählt ist aus der Gruppe, bestehend aus VLA-1, VLA-2, VLA-3, VLA-4, VLA-5, VLA-6, Leucam, Mac-1, LFA-1, gp150.95, CD41a, CD49 und CD51.

56. Therapeutische Zusammensetzung gemäss Anspruch 53, wobei das Mitglied der Immunoglobulin-Superfamilie ausgewählt ist aus der Gruppe, bestehend aus ICAM-1, ICAM-2 oder ICAM-3, VCAM, NCAM und PECAM.

57. Therapeutische Zusammensetzung gemäss Anspruch 53, wobei das Wirtsadhäsionsmolekül ein Guanosintriphosphat-Bindungsprotein ist.

58. Therapeutische Zusammensetzung gemäss Anspruch 57, wobei das Guanosintriphosphat-Bindungsprotein ausgewählt ist aus der Gruppe, bestehend aus Rho, Ras, Rac, Dcd42, Rab, Ran und Arf.

59. Vaccin gemäss Anspruch 1, formuliert zur mucosalen Zufuhr, wobei das Vaccin oral oder intranasal verabreicht wird.

60. Vaccin gemäss Anspruch 1, wobei das pathogene Adhesinmolekül ausgewählt ist aus der Gruppe, bestehend aus Proteinen, Glycoproteinen, Glycolipiden, Kohlenhydraten, Lectinen, Selectinen, Integrinen und funktionellen Äquivalenten davon.

## Revendications

1. Vaccin comprenant un support acceptable sur le plan pharmaceutique et une molécule d'adhésine pathogène isolée ou un fragment immunogène de celle-ci, dans lequel la molécule d'adhésine pathogène ou le fragment immunogène de celle-ci se lie de façon spécifique à une molécule d'adhésion d'une cellule hôte ou de la matrice extracellulaire, permettant ainsi audit agent pathogène de circuler dans le tissu hôte, et dans lequel ledit vaccin induit une réponse immunitaire thérapeutiquement efficace contre l'agent pathogène.

2. Vaccin selon la revendication 1, dans lequel ladite molécule d'adhésine pathogène ou ledit fragment immunogène de celle-ci se lie de façon spécifique à ladite molécule d'adhésion d'une cellule hôte ou de la matrice extracellulaire dans des conditions de cisaillement in vitro.

3. Vaccin selon la revendication 2, dans lequel lesdites conditions de cisaillement sont choisies parmi le groupe constitué de conditions de cisaillement physiologiques existant, typiquement, au niveau : (1) du système vasculaire ; (2) du système respiratoire ; (3) du tube digestif ; et (4) des voies urinaires.

4. Vaccin selon la revendication 1, dans lequel ladite cellule hôte est choisie parmi le groupe constitué des leucocytes, des cellules endothéliales, des cellules épithéliales et des cellules du système nerveux .

5. Vaccin selon la revendication 1, dans lequel ladite molécule d'adhésine pathogène se comporte, fonctionnellement parlant, comme un ligand pour ladite molécule d'adhésion hôte.

6. Vaccin selon la revendication 1, dans lequel ladite molécule d'adhésion hôte est un récepteur pour un ligand hôte choisi parmi le groupe constitué des lectines de type C, des sélectines, des intégrines, des membres de la superfamille des immunoglobulines et des cytokines.

7. Vaccin selon la revendication 6, dans lequel lesdites sélectines sont choisies parmi le groupe constitué des sélectines E, L et P.

8. Vaccin selon la revendication 6, dans lequel ladite intégrine est choisie parmi le groupe constitué des éléments VLA-1, VLA-2, VLA-3, VLA-4, VLA-5, VLA-6, leucam, Mac-1, LFA-1, gp150.95, CD41a, CD49 et CD51.

9. Vaccin selon la revendication 6, dans lequel ledit membre de la superfamille des immunoglobulines est choisi parmi le groupe constitué des éléments ICAM-1, ICAM-2 ou ICAM-3, VCAM, NCAM et PECAM.

10. Vaccin selon la revendication 1, dans lequel ladite molécule d'adhésion hôte est choisie parmi le groupe constitué de protéines, de glycoprotéines, de glycolipides et de glucides.

11. Vaccin selon la revendication 1, dans lequel ladite molécule d'adhésine pathogène se lie à un ligand glucidique choisi parmi le groupe constitué de résidus de l'acide N-acétylneuraminique, de l'acide sialique, de la N-acétylglucosamine, de la N-acétylgalactosamine, de la glucosamine, de la galactosamine, du galactose, du mannose, du fucose et du lactose.

12. Vaccin selon la revendication 1, dans lequel ladite molécule d'adhésion hôte est une protéine liant la guanosine triphosphate.

13. Vaccin selon la revendication 1, mais dans lequel ladite protéine liant la guanosine triphosphate est choisie parmi le groupe constitué des éléments Rho, Ras, Rac, Dcd42, Rab, Ran et Arf.

14. Vaccin selon la revendication 1, dans lequel ladite cellule hôte est choisie parmi le groupe constitué des cellules endothéliales stimulées par la cytokine et des cellules endothéliales exprimant ICAM-1, CAM-1, MAdCAM-1 et PNAd-1.

15. Vaccin selon la revendication 1, dans lequel ledit agent pathogène est choisi parmi le groupe constitué de virus, de bactéries, de protozoaires et de champignons.

16. Vaccin selon la revendication 15, dans lequel ledit agent pathogène est un virus.

17. Vaccin selon la revendication 15, dans lequel ledit agent pathogène est une bactérie.

18. Vaccin selon la revendication 15, dans lequel ledit agent pathogène est un protozoaire.

19. Vaccin selon la revendication 15, dans lequel ledit agent pathogène est un champignon.

20. Vaccin selon la revendication 15, dans lequel l'agent pathogène est un agent pathogène intestinal choisi parmi le groupe constitué de *Vibrio cholerae, Escherichia coli* uropathogène, *E. coli* entéro-hémorragique, *E. coli* entéro-pathogène, des Salmonella, des *Shigella,* des *Pseudomonas,* des Proteus, *Klebsiella* pneumoniae, *Aerobacter areogenes* et *Heliobacter pylori*.

21. Vaccin selon la revendication 15, dans lequel ledit agent pathogène est choisi parmi le groupe des cellules sanguines constitué de *Plasmodium berghei,* de *Plasmodium falciparum,* des Brucella, de *Neisseia meningitidis,* des *Staphylococcus,* de Pasteurella *pestis,* de *Leishmania,* de *Trypanosoma* et de Pasteurella *tularensis.*

22. Vaccin selon la revendication 15, dans lequel ledit agent pathogène est choisi parmi le groupe constitué de *Mycobacterium tuberculosis,* de Legionella, des *Staphylococcus,* des *Streptococus,* de Bordetella *pertussis,* de *Pasteurella pestis,* de *Hemophilus influenzae* et de Cprumebacterum *diphtheriae.*

23. Vaccin selon la revendication 15, dans lequel ledit agent pathogène est choisi parmi le groupe des parasites fongiques constitué de *Blastomyces, d'Aspergillus,* de *Cryptococcus,* de Candida, de *Histoplasma,* de *Coccidioides* et de *Phycomycetes.*

24. Vaccin selon la revendication 15, dans lequel ledit agent pathogène est choisi parmi le groupe des parasites intestinaux constitué d'Entamoeba *histolytica,* de *Giardia* lamblia'et de *Cryptosporidium.*

25. Vaccin selon la revendication 15, dans lequel ledit agent pathogène est parmi le groupe des agents pathogènes de l'appareil uro-génital constitué de *Neisseria gonorrhoeae, de Chlamydia,* de *Treponema* Pallidium, de *Trichomonas* vaginales et de *Tritrichomonas foetus*.

26. Vaccin selon la revendication 15, dans lequel ledit agent pathogène est choisi parmi le groupe de virus constitué des virus de l'influenza, A, de *l'influenza* B, de l'influenza *C,* de la rougeole, des *oreillons,* de l'adénovirus, du *rhinovirus,* du *poliovirus,* du virus de l'hépatite, de l'hantavirus, de *l'herpèsvirus,* du virus de la rubéole, du *virus de l'immunodéficience* humaine *(VIH)* et du virus *Coxsackie.*

27. Vaccin selon la revendication 1, dans lequel ladite cellule hôte est choisie parmi le groupe des cellules de l'appareil respiratoire constitué des macrophages alvéolaires et des cellules endothéliales et épithéliales du rhino-pharynx et des alvéoles.

28. Vaccin selon la revendication 1, dans lequel ledit vaccin comprend, en outre, des fractions peptidiques de la région adhésive de 1'3-oligomère d'une exotoxine choisie parmi un groupe constitué de l'exotoxine de *Corynebacterium* diptheriae, de la toxine de *Bordetella pertussis,* de la toxine de *Shigella dysenteriae* (type I), de la toxine de *Salmonella typhimurium,* de la toxine de *Vibrio cholerae,* de la vérotoxine entéro-hémorragique *d'Escherichia coli,* de l'entéro-toxine entéro-pathogène *d'Escherichia coli,* de l'exotoxine de *Pseudomonas aeruginosa,* de l'exotoxine de *Clostridium* tetani et de l'exotoxine de *Clostridium botulinum.*

29. Vaccin selon la revendication 1, dans lequel ledit vaccin comprend, en outre, les fractions peptidiques de la région adhésive de la lectine sur les fimbrias présentes sur des microbes choisis parmi le groupe constitué *d'Escherichia coli,* de *Neisseria gonorrhoeae,de Neisseria* meningitidis,de *Salmonella* typhi, de *Salmonella typhimurium,* d'autres salmonelles, de Pseudomonas *aeruginosa* et de *Yersinia* enterocolitica.

30. Vaccin selon la revendication 1, dans lequel ledit vaccin comprend, en outre, les fractions peptidiques des molécules d'adhésion des glycoprotéines présentes à la surface cellulaire des microbes choisis parmi un groupe constitué *d'Escherichia coli,de Yersinia pseudotuberculosis, d'Helicobacter pylori,* de *Vibrio cholera,* de *Salmonella* typhi, de salmonella *typhimurium,* de Shigella *dysenteriae,* de *Leishmania,* de *Giardia lambia,* de *Entamoeba* histolytica, de *Candida* albicans et de Harnia *alvae.*

31. Vaccin selon la revendication 1, dans lequel ledit vaccin comprend, en outre, la fraction peptidique des molécules d'adhésion des glycoprotéines qui se lient aux ligands de l'acide sialique au niveau des cellules du système nerveux.

32. Vaccin selon la revendication 1, dans lequel ledit vaccin comprend les fractions peptidiques d'une molécule d'adhésion des glycoprotéines microbiennes choisie parmi le groupe constitué de *Neisseria meningitidis* et *Escherichia coli* K1.

33. Vaccin selon la revendication 1, comprenant en outre un dispositif de délivrance présentant ladite molécule d'adhésion pathogène ou un fragment immunogène de celle-ci.

34. Vaccin selon la revendication 33, dans lequel ledit dispositif de délivrance est choisi parmi le groupe constitué des bactériophages, d'un vecteur vivant, de liposomes, des bactériophages M13, du virus de la mosaïque de la dolique, des gels d'alginate, des conjugués peptidiques et des glycoconjugués.

35. Vaccin selon la revendication 34, dans lequel ledit vecteur vivant appartient au genre *Salmonella* ou *Shigella* ou est un adénovirus.

36. Vaccin comprenant un support acceptable sur le plan pharmaceutique et une molécule d'adhésine pathogène isolée ou un fragment immunogène de celle-ci, dans lequel la molécule d'adhésine pathogène se lie de façon spécifique à une molécule d'adhésion sur la cellule hôte ou une matrice extracellulaire dans des conditions de cisaillement physiologique in vitro, et dans lequel ledit vaccin induit une réponse immunitaire thérapeutiquement efficace contre l'agent pathogène.

37. Vaccin comportant un support acceptable sur le plan pharmaceutique et une copie de molécule d'adhésine pathogène isolée, dans lequel ladite copie induit une réponse immunitaire thérapeutiquement efficace contre l'agent pathogène.

38. Procédé d'obtention d'un vaccin contre un agent pathogène comportant les étapes consistant à :
(a) sélectionner une molécule d'adhésine pathogène ou des fragments de celle-ci se liant de façon spécifique à une molécule d'adhésion sur une cellule hôte ou une matrice extracellulaire ;
(b) développer un ou plusieurs anticorps monoclonaux (mAbs) dirigés contre au moins une région de la molécule d'adhésine isolée ou des fragments de celle-ci ;
(c) isoler des déterminants antigéniques liés par lesdits mAbs ; et
(d) trier lesdits déterminants antigéniques sélectionnés pour identifier une molécule induisant une réponse immunitaire thérapeutiquement efficace contre l'agent pathogène.

39. Procédé selon la revendication 38, dans lequel ladite étape de sélection est réalisée au moyen d'une réaction de cisaillement en utilisant des cellules cibles exprimant la molécule d'adhésion hôte.

40. Procédé selon la revendication 38, comprenant en outre une étape consistant à analyser la spécificité et les propriétés d'inhibition des mAbs pour les interactions agent pathogène/cellule hôte par une réaction de cisaillement.

41. Procédé selon la revendication 38, dans lequel ladite étape consistant à isoler des déterminants antigéniques comprend l'examen d'une bibliothèque de bactériophages de visualisation pour identifier les copies de la molécule d'adhésine pathogène qui se lient aussi à un ou à plusieurs desdits anticorps monoclonaux.

42. Kit diagnostic comportant un anticorps monoclonal spécifique à une molécule d'adhésine pathogène, dans lequel ladite molécule est capable de se lier de façon spécifique à une molécule d'adhésion sur une cellule hôte ou une matrice extracellulaire dans des conditions de cisaillement in *vitro.*

43. Kit diagnostic selon la revendication 42, dans lequel ladite molécule d'adhésine pathogène se comporte, sur le plan fonctionnel, comme un ligand pour ladite molécule d'adhésion hôte.

44. Kit diagnostic selon la revendication 42, comprenant en outre des billes superparamagnétiques recouvertes desdits anticorps monoclonaux.

45. Kit diagnostic comprenant un peptide ou un oligopeptide se comportant, sur le plan fonctionnel, comme la fraction adhésive d'une molécule d'adhésine pathogène, dans lequel ladite molécule se lie de façon spécifique à une molécule d'adhésion sur une cellule hôte ou une matrice extracellulaire dans des conditions de cisaillement *in vitro.*

46. Composition pour test diagnostic comprenant une matrice glycolipidique montrant des molécules d'adhésion hôtes présentant un résidu choisi parmi le groupe constitué des résidus de l'acide N-acétylneuraminique, de l'acide sialique, de la N-acétylglucosamine, de la N-acétylgalactosamine, de la glucosamine, de la galactosamine, du galactose, du mannose, du fucose et du lactose ; dans lequel ladite molécule d'adhésion hôte se lie de façon spécifique à une molécule d'adhésine pathogène dans des conditions de cisaillement *in vitro.*

47. Composition thérapeutique comprenant un support acceptable sur le plan pharmaceutique et une molécule d'adhésine pathogène isolée ou un fragment de celle-ci, dans laquelle ladite molécule d'adhésine pathogène ou ledit fragment se lie de façon spécifique à une molécule d'adhésion d'une cellule hôte ou de la matrice extracellulaire dans des conditions de cisaillement *in vitro.*

48. Composition thérapeutique selon la revendication 46, dans laquelle ladite composition inhibe un ou plusieurs des événements suivants associés à l'infection par l'agent pathogène de l'hôte infecté, lesdits événements étant choisis parmi le groupe constitué des événements suivants : (1) reconnaissance par l'agent pathogène de cellules hôtes spécifiques ou d'une matrice extracellulaire ; (2) fixation, uniquement en cas de cisaillement, de l'agent pathogène sur les cellules hôtes ou la matrice extracellulaire ; (3) adhésion, uniquement en cas d'activation, de l'agent pathogène ; (4) transduction du signal par l'entremise de l'agent pathogène ; (5) migration trans-endothéliale de l'agent pathogène ; (6) traversée par l'agent pathogène de l'épithélium ; (7) colonisation par l'agent pathogène ; et (8) liaison d'une toxine produite par l'agent pathogène aux cellules hôtes ou à la matrice extracellulaire.

49. Composition thérapeutique selon la revendication 48, dans laquelle ladite composition se lie aux membres de la famille des sélectines des molécules d'adhésion hôtes.

50. Composition thérapeutique selon la revendication 48, dans laquelle ladite composition se lie aux membres de la superfamille des immunoglobulines des molécules d'adhésion hôtes.

51. Composition thérapeutique selon la revendication 48, dans laquelle ladite composition se lie aux membres de la famille des intégrines des molécules d'adhésion hôtes.

52. Composition thérapeutique selon la revendication 48, dans laquelle ladite molécule d'adhésine pathogène se comporte sur le plan fonctionnel comme un ligand pour ladite molécule d'adhésion hôte.

53. Composition thérapeutique selon la revendication 52, dans laquelle ladite molécule d'adhésion hôte constitue un récepteur pour un ligand hôte choisi parmi le groupe constitué de sélectines, d'intégrines, de membres de la superfamille des immunoglobulines, de cytokines et de protéines liant la guanosine triphosphate.

54. Composition thérapeutique selon la revendication 53, dans laquelle ladite sélectine est choisie parmi le groupe constitué des sélectines E, L et P.

55. Composition thérapeutique selon la revendication 53, dans laquelle ladite intégrine est choisie parmi le groupe constitué des éléments VLA-1, VLA-2, VLA-3, VLA-4, VLA-5, VLA-6, leucam, Mac-1, LFA-1, gp150.95, CD41a, CD49 et CD51.

56. Composition thérapeutique selon la revendication 53, dans laquelle ledit membre de la superfamille des immunoglobulines est choisi parmi le groupe constitué des éléments ICAM-1, ICAM-2 ou ICAM-3, VCAM, NCAM et PECAM.

57. Composition thérapeutique selon la revendication 53, dans laquelle ladite molécule d'adhésion hôte est une protéine liant la guanosine triphosphate.

58. Composition thérapeutique selon la revendication 57, dans laquelle ladite protéine liant la guanosine triphosphate est choisie parmi le groupe constitué des éléments Rho, Ras, Rac, Dcd42, Rab, Ran et Arf.

59. Vaccin selon la revendication 1, formulé pour une délivrance par voie muqueuse, dans lequel le vaccin est administré par voie orale ou intranasale.

60. Vaccin selon la revendication 1, dans lequel ladite molécule d'adhésine pathogène est choisie parmi le groupe constitué des protéines, des glycoprotéines, des glycolipides, des glucides, des lectines, des sélectines, des intégrines et des équivalents fonctionnels de ces derniers.
